# EUROPEAN PATENT APPLICATION

(11) **EP 4 252 779 A2**
(43) Date of publication of application: **04.10.2023**
(21) Application number: 23179978.4
(22) Date of filing: 28.06.2018
(51) Int. Cl.: A61K 47/42

(54) **DISSOLVING FILM FOR DELIVERY OF A CLOSTRIDIAL DERIVATIVE**

(30) Priority: 30.06.2017 US 201762527212 P
(62) Divisional of application: 18743268.7
(71) Applicant: Allergan, Inc., Irvine, CA 92612 (US)
(72) Inventor: Brin, Mitchell F., Newport Beach, CA, 92660 (US); Hughes, Patrick M., Aliso Viejo California, 92656 (US); Lu, Guang Wei, Irvine California, 92612 (US)
(74) Representative: Hoffmann Eitle

(57) **Abstract**

A biodegradable film comprising a therapeutically effective amount of a Clostridial neurotoxin is described. Methods for treating rhinitis and for treating a symptom of rhinitis by administering the biodegradable film to the nasal cavity are also described.

## Description

### CROSS-REFERENCE TO RELATED APPLICATIONS

This application claims the benefit of U.S. Provisional Application Ser. No. 62/527,212, filed June 30, 2017, which is hereby incorporated by reference in its entirety.

### TECHNICAL FIELD

The subject matter described herein relates to a dissolving film for delivery of an active agent to a mucosal surface, for local or systemic delivery of the agent. In particular, a dissolving film that comprises a Clostridial toxin as the active ingredient is described.

### BACKGROUND

The anaerobic, gram positive bacterium *Clostridium botulinum* produces a potent polypeptide neurotoxin, botulinum toxin, which causes a neuroparalytic illness in humans and animals referred to as botulism. The spores of Clostridium botulinum are found in soil and can grow in improperly sterilized and sealed food containers of home based canneries, which are the cause of many of the cases of botulism. The effects of botulism typically appear 18 to 36 hours after eating the foodstuffs infected with a *Clostridium botulinum* culture or spores. The botulinum toxin can apparently pass unattenuated through the lining of the gut and shows a high affinity for cholinergic motor neurons. Symptoms of botulinum toxin intoxication can progress from difficulty walking, swallowing and speaking to paralysis of the respiratory muscles and death.

Commercially available botulinum toxin containing pharmaceutical compositions include BOTOX^{®} (Botulinum toxin type A neurotoxin complex with human serum albumin and sodium chloride) (Allergan, Inc., Irvine, CA), DYSPORT^{®} (Clostridium botulinum type A toxin haemagglutinin complex with human serum albumin and lactose in the formulation) (Ipsen Limited, Berkshire, U.K.) as a powder to be reconstituted with 0.9% sodium chloride before use), and MYOBLOC^{™} (an injectable solution comprising botulinum toxin type B, human serum albumin, sodium succinate, and sodium chloride at about pH 5.6 (Solstice Neurosciences, Inc., South San Francisco, CA). More recently, non-protein-based (animal protein-free) botulinum toxin containing pharmaceutical compositions have been described (see, *e.g.* U.S. Published Application No. 2012/0207787, the contents of which are incorporated by reference herein).

Botulinum toxin is a large protein for incorporation into a pharmaceutical formulation (the molecular weight of the botulinum toxin type A complex is 900 kD) and is inherently fragile and labile. The size of the toxin complex makes it much more friable and labile than smaller, less complex proteins, thereby compounding the formulation and handling difficulties if botulinum toxin stability is to be maintained. Hence, a botulinum toxin stabilizer must be able to interact with the toxin in a manner which does not denature, fragment or otherwise detoxify the toxin molecule or cause disassociation of the non-toxin proteins present in the toxin complex.

Botulinum toxins have been used for various therapeutic and cosmetic purposes including treating cervical dystonia, blepharospasm, strabismus, spasticity, headache, hyperhidrosis, overactive bladder, rhinitis, bruxism, enlarged prostate, achalasia, anismus, sphincter of Oddi malfunction, acne, tremors, juvenile cerebral palsy, and facial wrinkles. A system for delivering botulinum toxin in a foam to a nasopharyngeal mucosa target is described in U.S. Patent No. 9,327,104 highlights the challenges in delivering botulinum toxin. Inadvertent or incorrect distribution of the toxin limits the ability to deliver a toxin.

As the most lethal known biological product, exceptional safety, precision, and accuracy are called for at all steps of the formulation of a botulinum toxin containing pharmaceutical composition.

Typically, therapeutic use of a botulinum toxin is by subcutaneous or intramuscular injection of an aqueous solution of a botulinum toxin type A or B. A repeat injection can be administered every 2-4 months to maintain therapeutic efficacy of the toxin (*i.e*. a reduction of muscle spasm at or in the vicinity of the injection site). Each administration of a dose of a botulinum toxin to a patient therefore requires the patient to present himself to his physician at regular intervals. Unfortunately, patients can forget or be unable to attend appointments and physician schedules can make regular, periodic care over a multiyear period difficult to consistently maintain. Additionally, the requirement for 3-6 toxin injections per year on an ongoing basis increases the risk of infection or of misdosing the patient.

Fast-dissolving drug delivery systems have been increasingly utilized due to their ease of use, especially for pediatric and geriatric patients. Depending on the application site, fast-dissolving films also offer the advantage of direct absorbance of an active agent to systemic circulation and avoidance of gastrointestinal degradation and first pass effects. Current dissolvable film products are typically used for oral administration or transmucosal delivery. Film products have also been developed for other routes of administration such as vaginal contraceptive films and teeth whitening strips.

It would be beneficial to provide a therapeutic dosage of a Clostridial toxin that is safe and/or effective for a subject to administer without the presence of a physician. It would also be beneficial to provide a composition for non-invasive or less invasive delivery of a Clostridial toxin. It would additionally be beneficial to provide a therapeutic dosage of a Clostridial toxin for local effect.

### BRIEF SUMMARY

The following aspects and embodiments thereof described and illustrated below are meant to be exemplary and illustrative, not limiting in scope.

In a first aspect, a method of treating rhinitis or for treating or alleviating one or more symptoms of rhinitis with a Clostridial toxin active ingredient or a botulinum toxin active ingredient is provided. In one embodiment, the method comprises administering a film comprising a therapeutically effective amount of a Clostridial toxin active ingredient to a nasal cavity of a subject, thereby treating rhinitis or alleviating one or more symptoms of rhinitis. In embodiments, the film comprises a therapeutically effective amount of a biologically active Clostridial toxin active ingredient; at least one polymer selected from a hydrophilic polymer and a mucoadhesive polymer; and at least one of (a) at least one mucosal permeation enhancer; and (b) at least one dissolution controller. In embodiments, the film comprises at least about 0.0056-560 ng of the Clostridial toxin active ingredient. In some embodiments, the film dissolves at the administration site in less than about 10 minutes. In embodiments, the film dissolves at the administration site in about 10 seconds to about 10 minutes. In embodiments, the Clostridial toxin active ingredient is selected from the group consisting of botulinum toxin types A, B, C, D, E, F and G.

In embodiments, the at least one hydrophilic polymer is selected from the group consisting of polysaccharides, hydroxyalkyl starch derivatives, polyvinyl acetates, polyvinyl pyrrolidones, polyethylene glycols, and a poloxamer. In embodiments, the at least one mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers. In embodiments, the at least one mucosal permeation enhancer is selected from the group consisting of diethylene glycol monoethyl ether, glyceryl monooleate, and a cell penetrating peptide. In embodiments, the at least one dissolution controller is selected from the group consisting of sugars, glycerin, and propylene glycol. In one embodiment, the sugar is a monosaccharide, and in another embodiment, the sugar is a disaccharide.

In some embodiments, the method further comprises coating the tip of a delivery device with the dissolving film, inserting all or a portion of the delivery device into a nasal cavity of a subject and contacting a mucosal membrane of the subject with the film-coated tip in order to administer the dissolving film to the mucosal membrane.

In a second aspect, a biodegradable film comprising a Clostridial toxin active ingredient or a botulinum toxin active ingredient is described. In embodiments, the film comprises about 0.0056 to 560 ng of a therapeutically effective amount of a biologically active Clostridial toxin active ingredient; about 20 to 95% (w/w) of at least one polymer selected from a hydrophilic polymer and a mucoadhesive polymer; and at least one of a mucosal permeation enhancer and at least one dissolution controller. In some embodiments, the film comprises about 0.0001 to 15% (w/w) of at least one mucosal permeation enhancer. In some embodiments, the film comprises about 0.1 to 15 % (w/w) of at least one dissolution controller. In some embodiments, the film comprises at least one mucosal permeation enhancer and at least one dissolution controller. In embodiments, the Clostridial toxin active ingredient is selected from the group consisting of botulinum toxin types A, B, C, D, E, F and G.

In embodiments, the hydrophilic polymer is selected from the group consisting of polysaccharides, hydroxyalkyl starch derivatives, polyvinyl acetates, polyvinyl pyrrolidones, polyethylene glycols, and a poloxamer. In embodiments, the mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers. In some embodiments, the at least one mucosal permeation enhancer is selected from the group consisting of diethylene glycol monoethyl ether, glyceryl monooleate, and cell penetrating peptides. In embodiments, the dissolution controller is selected from the group consisting of sugars, glycerin, and propylene glycol.

In a further aspect, a dissolving film comprising a therapeutically effective amount of a Clostridial toxin active ingredient is described. In embodiments, the film further comprises at least one polymer selected from a hydrophilic polymer and a mucoadhesive polymer; at least one mucosal permeation enhancer; and at least one dissolution controller. In embodiments, the film comprises about 0.0056 to 560 ng of the Clostridial toxin active ingredient. In some embodiments, the Clostridial toxin active ingredient is selected from the group consisting of botulinum toxin types A, B, C, D, E, F and G.

In embodiments, the film comprises about 20 to 95% (w/w) of the at least one polymer. In some embodiments, the at least one polymer is selected from at least one of a hydrophilic polymer and a mucoadhesive polymer. In embodiments, the hydrophilic polymer is selected from the group consisting of polysaccharides, hydroxyalkyl starch derivatives, polyvinyl acetates, polyvinyl pyrrolidones, polyethylene glycols, and a poloxamer. In embodiments, the mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.

In embodiments, the film comprises about 0.1 to 15 % (w/w) of the at least one dissolution controller. In some embodiments, the at least one dissolution controller is selected from sugars, glycerin, and propylene glycol.

In embodiments, the film comprises about 0.0001 to 15% (w/w) of the at least one mucosal permeation enhancer. In some embodiments, the at least one mucosal permeation enhancer is selected from the group consisting of diethylene glycol monoethyl ether, glyceryl monooleate, and cell penetrating peptides.

Certain methods, compositions and formulations are described herein to provide a dissolvable film for delivery of an active agent such as a Clostridial toxin derivative. It will be appreciated that the film compositions as described below may refer to the film composition as a whole or to a layer of a film that includes the active agent. The film compositions may further comprise one or more excipients including, but not limited to, a disaccharide, a surfactant, and/or an antioxidant.

In addition to the exemplary aspects and embodiments described above, further aspects and embodiments will become apparent by reference to the examples and by study of the following descriptions.

Additional embodiments of the present methods and compositions, and the like, will be apparent from the following description, examples, and claims. As can be appreciated from the foregoing and following description, each and every feature described herein, and each and every combination of two or more of such features, is included within the scope of the present disclosure provided that the features included in such a combination are not mutually inconsistent. In addition, any feature or combination of features may be specifically excluded from any embodiment herein. Additional aspects and advantages of the present compositions, formulations, films, and methods are set forth in the following description and claims, particularly when considered in conjunction with the accompanying examples.

### DETAILED DESCRIPTION

### I. Definitions

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm.

The singular forms "a," "an," and "the" include plural referents unless the context clearly dictates otherwise. Thus, for example, reference to an "excipient" includes a single excipient as well as two or more of the same or different excipients, and the like.

"About" or "approximately" as used herein means within an acceptable error range for the particular value as determined by one of ordinary skill in the art, which will depend in part on how the value is measured or determined, (*i.e.,* the limitations of the measurement system). For example, "about" can mean within 1 or more than 1 standard deviations, per practice in the art. Where particular values are described in the application and claims, unless otherwise stated, the term "about" means within an acceptable error range for the particular value.

"Administration", or "to administer" means the step of giving (*i.e.* administering) a pharmaceutical composition or film composition to a subject, or alternatively a subject receiving a pharmaceutical or film composition. The pharmaceutical/film compositions disclosed herein can be locally or topically administered using a bioerosion or dissolvable film.

"Alleviating" or "relieving" means a reduction in the occurrence of a pain, of a headache, or of any symptom or cause of a condition or disorder such as rhinitis. Thus, alleviating includes some reduction, significant reduction, near total reduction, and total reduction.

"Animal protein free" means the absence of blood derived, blood pooled and other animal derived products or compounds. "Animal" means a mammal (such as a human), bird, reptile, fish, insect, spider or other animal species. "Animal" excludes microorganisms, such as bacteria. Thus, an animal protein free film composition can include a botulinum neurotoxin. For example, an "animal protein free" film composition means a film composition which is either substantially free or essentially free or entirely free of a serum derived albumin, gelatin and other animal derived proteins, such as immunoglobulins. An example of an animal protein free film composition is a film composition which comprises or which consists of a botulinum toxin (as the active ingredient) and a suitable polysaccharide as a stabilizer or excipient.

The term "dissolving film" or "dissolvable film" intends a sheet or layer comprised of a polymer, an active agent and one or more pharmaceutically acceptable excipients that when contacted with a physiological fluid, such as placed on a mucosal surface of a subject, dissolves the polymer and releases the active agent.

The terms "erosion", "bioerosion" and "erodible" refer to a process where a polymer or a polymer component of a film when contacted with a physiological fluid dissolves rather than degrades (i.e., breaking down of the polymer into small molecular weight materials by a chemical reaction) the polymer.

"Biological activity" describes the beneficial or adverse effects of a drug on living matter. When a drug is a complex chemical mixture, this activity is exerted by the substance's active ingredient but can be modified by the other constituents. Biological activity can be assessed as potency or as toxicity by an *in vivo* LD₅₀ or ED₅₀ assay, or through an *in vitro* assay such as, for example, cell-based potency assays as described in U.S. 2010/0203559 and U.S. 2010/0233802.

"Botulinum toxin" means a neurotoxin produced by Clostridium botulinum, as well as a botulinum toxin (or the light chain or the heavy chain thereof) made recombinantly by a non-Clostridial species. The phrase "botulinum toxin", as used herein, encompasses Botulinum toxin serotype A (BoNT/A), Botulinum toxin serotype B (BoNT/B), Botulinum toxin serotype C (BoNT/C), Botulinum toxin serotype D (BoNT/D), Botulinum toxin serotype E (BoNT/E), Botulinum toxin serotype F (BoNT/F), Botulinum toxin serotype G (BoNT/G), Botulinum toxin serotype H (BoNT/H), Botulinum toxin serotype X (BoNT/X), and mosaic Botulinum toxins and/or subtypes and variants thereof. "Botulinum toxin", as used herein, also encompasses a "modified botulinum toxin". Further "botulinum toxin" as used herein also encompasses a botulinum toxin complex, (for example, the 300, 600 and 900kDa complexes), as well as the neurotoxic component of the botulinum toxin (150 kDa) that is unassociated with the complex proteins.

"Clostridial toxin" refers to any toxin produced by a Clostridial toxin strain that can execute the overall cellular mechanism whereby a Clostridial toxin intoxicates a cell and encompasses the binding of a Clostridial toxin to a low or high affinity Clostridial toxin receptor, the internalization of the toxin/receptor complex, the translocation of the Clostridial toxin light chain into the cytoplasm and the enzymatic modification of a Clostridial toxin substrate. Non-limiting examples of Clostridial toxins include a Botulinum toxin like BoNT/A, a BoNT/B, a BoNT/Ci, a BoNT/D, a BoNT/E, a BoNT/F, a BoNT/G, a BoNT/H, a BoNT/X, a mosaic Botulinum toxins and/or subtypes and variants thereof, a Tetanus toxin (TeNT), a Baratii toxin (BaNT), and a Butyricum toxin (BuNT). The BoNT/C₂ cytotoxin and BoNT/C₃ cytotoxin, not being neurotoxins, are excluded from the term "Clostridial toxin." A Clostridial toxin disclosed herein includes, without limitation, naturally occurring Clostridial toxin variants, such as, *e.g.,* Clostridial toxin isoforms and Clostridial toxin subtypes; non-naturally occurring Clostridial toxin variants, such as, *e.g*., conservative Clostridial toxin variants, non-conservative Clostridial toxin variants, Clostridial toxin chimeric variants and active Clostridial toxin fragments thereof, or any combination thereof. A Clostridial toxin disclosed herein also includes a Clostridial toxin complex. As used herein, the term "Clostridial toxin complex" refers to a complex comprising a Clostridial toxin and non-toxin associated proteins (NAPs), such as, *e.g.,* a Botulinum toxin complex, a Tetanus toxin complex, a Baratii toxin complex, and a Butyricum toxin complex. Non-limiting examples of Clostridial toxin complexes include those produced by a *Clostridium botulinum,* such as, *e.g.,* a 900-kDa BoNT/A complex, a 500-kDa BoNT/A complex, a 300-kDa BoNT/A complex, a 500-kDa BoNT/B complex, a 500-kDa BoNT/C₁ complex, a 500-kDa BoNT/D complex, a 300-kDa BoNT/D complex, a 300-kDa BoNT/E complex, and a 300-kDa BoNT/F complex.

"Clostridial toxin active ingredient" or "Clostridial toxin active" refers to a molecule which contains any part of a Clostridial toxin that exerts an effect upon or after administration to a subject or patient. As used herein, the terms "Clostridial toxin active ingredient" and "Clostridial toxin active" encompass a Clostridial toxin complex comprising the approximately 150-kDa Clostridial toxin and other proteins collectively called non-toxin associated proteins (NAPs), the approximately 150-kDa Clostridial toxin alone, or a modified Clostridial toxin, such as, *e.g.,* a re-targeted Clostridial toxins.

A "Clostridial toxin derivative" as used herein refers to a "Clostridial toxin" as described above as well as a modified, recombinantly produced, or fragment of a Clostridial toxin.

"Effective amount" as applied to the biologically active ingredient means that amount of the ingredient which is generally sufficient to effect a desired change in the subject. For example, where the desired effect is a reduction in symptoms relating to rhinitis, an effective amount of the ingredient is that amount which causes at least a substantial reduction of one or more symptoms of rhinitis (*e.g*. nasal congestion, rhinorrhea, and itching of the mucus membranes) without resulting in significant toxicity.

"Effective amount" when used in reference to the amount of an excipient or specific combination of excipients added to a Clostridial toxin composition, refers to the amount of each excipient that is necessary to achieve the desired initial recovered potency of a Clostridial toxin active ingredient. In aspects of this embodiment, an effective amount of an excipient or combination of excipients results in an initial recovered potency of, *e.g.,* at least 10%, at least 20%, at least 30%, at least 40%, at least 50%, at least 60%, at least 70%, at least 80%, at least 90% or at least 100%. In other aspects of this embodiment, a therapeutically effective concentration of a Clostridial toxin active ingredient reduces a symptom associated with the aliment being treated by, *e.g.,* at most 10%, at most 20%,at most 30%, at most 40%, at most 50%, at most 60%, at most 70%, at most 80%, at most 90% or at most 100%.

"Heavy chain" means the heavy chain of a botulinum neurotoxin. It has a molecular weight of about 100kDa and can be referred to as the H chain, or as H.

He means a fragment (about 50kDa) derived from the H chain of a botulinum neurotoxin which is approximately equivalent to the carboxyl end segment of the H chain, or the portion corresponding to that fragment in the intact H chain. It is believed to be immunogenic and to contain the portion of the natural or wild type botulinum neurotoxin involved in high affinity, presynaptic binding to motor neurons.

H_{N} means a fragment (about 50kDa) derived from the H chain of a botulinum neurotoxin which is approximately equivalent to the amino end segment of the H chain, or the portion corresponding to that fragment in the intact in the H chain. It is believed to contain the portion of the natural or wild type botulinum neurotoxin involved in the translocation of the L chain across an intracellular endosomal membrane.

"Light chain" means the light chain of a Clostridial neurotoxin. It has a molecular weight of about 50kDa, and can be referred to as the L chain, L, or as the proteolytic domain (amino acid sequence) of a botulinum neurotoxin.

LH_{N} or L-H_{N} means a fragment derived from a Clostridial neurotoxin that contains the L chain, or a functional fragment thereof coupled to the H_{N} domain. It can be obtained from the intact Clostridial neurotoxin by proteolysis, so as to remove or to modify the He domain.

"Film" means a thin layer of a composition or a drug delivery system providing release of an active agent including immediate release, controlled release (*e.g*., pulsatile or continuous) and/or sustained release of the active agent.

"Local administration" means direct administration of an active agent at or to the vicinity of a site on or within an animal body, at which site a biological effect of the pharmaceutical is desired, such as via, for example, topical administration. Local administration excludes systemic routes of administration, such as intravenous or oral administration. Topical administration is a type of local administration in which a pharmaceutical agent is applied to a patient's skin or mucous membrane (*e.g.* mouth or nasal cavity). Local administration may include sublingual, buccal, and nasal administration.

"Modified botulinum toxin" means a botulinum toxin that has had at least one of its amino acids deleted, modified, or replaced, as compared to a native botulinum toxin. Additionally, the modified botulinum toxin can be a recombinantly produced neurotoxin, or a derivative or fragment of a recombinantly made neurotoxin. A modified botulinum toxin retains at least one biological activity of the native botulinum toxin, such as, the ability to bind to a botulinum toxin receptor, or the ability to inhibit neurotransmitter release from a neuron. One example of a modified botulinum toxin is a botulinum toxin that has a light chain from one botulinum toxin serotype (such as serotype A), and a heavy chain from a different botulinum toxin serotype (such as serotype B). Another example of a modified botulinum toxin is a botulinum toxin coupled to a neurotransmitter.

"Mutation" means a structural modification of a naturally occurring protein or nucleic acid sequence. For example, in the case of nucleic acid mutations, a mutation can be a deletion, addition or substitution of one or more nucleotides in the DNA sequence. In the case of a protein sequence mutation, the mutation can be a deletion, addition or substitution of one or more amino acids in a protein sequence. For example, a specific amino acid comprising a protein sequence can be substituted for another amino acid, for example, an amino acid selected from a group which includes the amino acids alanine, asparagine, cysteine, aspartic acid, glutamic acid, phenylalanine, glycine, histidine, isoleucine, lysine, leucine, methionine, proline, glutamine, arginine, serine, threonine, valine, tryptophan, tyrosine or any other natural or non-naturally occurring amino acid or chemically modified amino acids. Mutations to a protein sequence can be the result of mutations to DNA sequences that when transcribed, and the resulting mRNA translated, produce the mutated protein sequence. Mutations to a protein sequence can also be created by fusing a peptide sequence containing the desired mutation to a desired protein sequence.

"Nasal administration" means direct or local administration of the active agent to the nasal cavity of a subject, especially to the nasal mucosa. Nasal administration includes administration to achieve local or systemic effects. Nasal administration may include administration of the active agent to any one or more of the nasal vestibule, the nasal atrium, the olfactory region, the respiratory region, and including the turbinates.

"Patient" means a human or non-human subject receiving medical or veterinary care. Accordingly, the compositions as disclosed herein can be used in treating any animal, such as, for example, mammals, or the like.

"Pharmaceutical composition" means a composition comprising an active pharmaceutical ingredient, such as, for example, a Clostridial toxin active ingredient such as a botulinum toxin, and at least one additional ingredient, such as, for example, a stabilizer or excipient or the like. A pharmaceutical composition is therefore a formulation which is suitable for diagnostic or therapeutic administration to a subject, such as a human patient. The pharmaceutical composition can be, for example, included in a thin film or membrane or used to form the thin film or membrane. The terms "pharmaceutical composition" and "film composition" are used interchangeably herein except where indicated otherwise by context.

"Pharmacologically acceptable excipient" is synonymous with "pharmacological excipient" or "excipient" and refers to any excipient that has substantially no long term or permanent detrimental effect when administered to mammal and encompasses compounds such as, *e.g.*, stabilizing agent, a bulking agent, a cryo-protectant, a lyo-protectant, an additive, a vehicle, a carrier, a diluent, or an auxiliary. An excipient generally is mixed with an active ingredient, or permitted to dilute or enclose the active ingredient and can be a solid, semi-solid, or liquid agent. It is also envisioned that a pharmaceutical composition comprising a Clostridial toxin active ingredient can include one or more pharmaceutically acceptable excipients that facilitate processing of an active ingredient into pharmaceutically acceptable film compositions. Insofar as any pharmacologically acceptable excipient is not incompatible with the Clostridial toxin active ingredient, its use in pharmaceutically acceptable compositions is contemplated. Non-limiting examples of pharmacologically acceptable excipients can be found in, *e.g.,* Pharmaceutical Dosage Forms and Drug Delivery Systems (Howard C. Ansel et al., eds., Lippincott Williams & Wilkins Publishers, 7th ed. 1999); Remington: The Science and Practice of Pharmacy (Alfonso R. Gennaro ed., Lippincott, Williams & Wilkins, 20th ed. 2000); Goodman & Gilman's The Pharmacological Basis of Therapeutics (Joel G. Hardman et al., eds., McGraw-Hill Professional, 10th ed. 2001); and Handbook of Pharmaceutical Excipients (Raymond C. Rowe et al., APhA Publications, 4th edition 2003), each of which is hereby incorporated by reference in its entirety.

The constituent ingredients of a delivery system can be included in a single composition or component (that is, the film or membrane comprises all of the constituent ingredients) or as a two-component system that contains one or more additional ingredients not present in the pharmaceutical composition of the film or membrane. A two-component system can provide several benefits, including that of allowing incorporation of ingredients which are not sufficiently compatible for long-term shelf storage with the first component of the two component system. Other ingredients, which may not be compatible with a Clostridial toxin active ingredient or a botulinum toxin or other ingredients for long periods of time, can be incorporated in this manner; that is, added in a second vehicle at the approximate time of use.

"Polysaccharide" means a polymer of more than two saccharide molecule monomers. The monomers can be identical or different.

"Stabilizing agent", "stabilization agent" or "stabilizer" means a substance that acts to stabilize a Clostridial toxin active ingredient such that the potency of the pharmaceutical composition is increased relative to an unstabilized composition.

"Stabilizers" can include excipients, and can include protein and non-protein molecules.

"Stabilizing", "stabilizes", or "stabilization" mean that an active pharmaceutical ingredient ("API") retains at least about 20% and up to about 100% of its biological activity (which can be assessed as potency or as toxicity by an in vivo LD₅₀ or ED₅₀ measure) in the presence of a compound which is stabilizing, stabilizes or which provides stabilization to the API as compared to the potency or toxicity of the biologically active Clostridial toxin active ingredient or botulinum toxin prior to being incorporated into the pharmaceutical composition.

"Substantially" or "essentially" means nearly totally or completely, for instance, 90-95% or greater of some given quantity.

"Substantially free" means nearly totally or completely absent of some given quantity such as being present at a level of less than about 1-5% of some given quantity. In some embodiments, "substantially free" means presence at a level of less than or equal to 1-5% by weight of the pharmaceutical composition.

"Therapeutic formulation" means a formulation can be used to treat and thereby alleviate a disorder or a disease, such as, for example, a disorder or a disease characterized by hyperactivity (*i.e*. spasticity) of a peripheral muscle.

"TEM" as used herein, is synonymous with "Targeted Exocytosis Modulator" or "retargeted endopeptidase." Generally, a TEM comprises an enzymatic domain from a Clostridial toxin light chain, a translocation domain from a Clostridial toxin heavy chain, and a targeting domain. The targeting domain of a TEM provides an altered cell targeting capability that targets the molecule to a receptor other than the native Clostridial toxin receptor utilized by a naturally-occurring Clostridial toxin. This re-targeted capability is achieved by replacing the naturally-occurring binding domain of a Clostridial toxin with a targeting domain having a binding activity for a non-Clostridial toxin receptor. Although binding to a non-Clostridial toxin receptor, a TEM undergoes all the other steps of the intoxication process including internalization of the TEM/receptor complex into the cytoplasm, formation of the pore in the vesicle membrane and di-chain molecule, translocation of the enzymatic domain into the cytoplasm, and exerting a proteolytic effect on a component of the SNARE complex of the target cell.

"Topical administration" excludes systemic administration of the neurotoxin. In other words, and unlike conventional therapeutic transdermal methods, topical administration of a Clostridial toxin active ingredient or botulinum toxin does not result in significant amounts, such as the majority of, the neurotoxin passing into the circulatory system of the patient.

"Transmucosal administration" means administration to the mucosal surface including sublingual, buccal and other oral tissue, and other mucosal surfaces including nasal, vaginal, rectal, ocular, gastrointestinal, and urethral. Transmucosal may provide systemic and/or local effect.

"Treating" means to alleviate (or to eliminate) at least one symptom of a condition or disorder, such as, for example, rhinitis, wrinkles, spasticity, depression, pain (such as, for example, headache pain), bladder overactivity, or the like, either temporarily or permanently.

"Variant" means a Clostridial neurotoxin, such as wild-type botulinum toxin serotype A, B, C, D, E, F or G, that has been modified by the replacement, modification, addition or deletion of at least one amino acid relative to wild-type botulinum toxin, which is recognized by a target cell, internalized by the target cell, and catalytically cleaves a SNARE (SNAP (Soluble NSF Attachment Protein) Receptor) protein in the target cell. An example of a variant neurotoxin component can comprise a variant light chain of a botulinum toxin having one or more amino acids substituted, modified, deleted and/or added. This variant light chain may have the same or better ability to prevent exocytosis, for example, the release of neurotransmitter vesicles. Additionally, the biological effect of a variant may be decreased compared to the parent chemical entity. For example, a variant light chain of a botulinum toxin type A having an amino acid sequence removed may have a shorter biological persistence than that of the parent (or native) botulinum toxin type A light chain.

### II. Film Compositions

In embodiments, a film is provided for administration of a Clostridial toxin active ingredient and/or a Clostridial toxin active ingredient or botulinum toxin active ingredient. The film is formulated to maintain position after administration and to deliver Clostridial toxin active ingredient at the administration site. In embodiments, the film is comprised of a therapeutically effective amount of a biologically active Clostridial or botulinum toxin, at least one polymer that dissolves in an aqueous environment and that facilitates adhesion at the administration site. In embodiments, the film includes one or more water-soluble polymers that dissolve at the administration site. In embodiments, the film includes, additionally or instead of the one or more water-soluble polymers, one or more bioadhesive and/or mucoadhesive polymers. It will be appreciated that the film may include one or more polymers that dissolve at the administration site and that are bioadhesive and/or mucosadhesive, which may be used interchangeably unless otherwise indicated by context. The film may further include one or more excipients including, but not limited to, dissolution controllers and permeation enhancers.

In some embodiments, the film is a mucoadhesive film suitable for administration of the active agent to a mucosal membrane or mucosa. Mucosal membranes include the mucous membranes of the mouth (buccal cavity), nasal cavity, rectum, vagina, and gastrointestinal membranes.

In embodiments, the film completely or substantially dissolves within about 10 seconds to about 10 minutes after placement at an administration site. In embodiments, the film completely or substantially dissolves or erodes within about 30 seconds to about 10 minutes, within about 1 to about 10 minutes, within about 5 to about 10 minutes, within about 10 seconds to about 5 minutes, within about 30 seconds to about 5 minutes, within about 1 to about 5 minutes, within about 10 seconds to about 1 minute, within about 30 seconds to about 1 minute, and within about 10 to about 30 seconds. The dissolution time for the film may be adjusted or selected based on the polymer and/or addition of one or more dissolution controllers that may increase or decrease the dissolution rate of the film. The dissolution time for the film may also, or in addition, be adjusted or selected based on the thickness and/or size of the film.

In some embodiments, the film comprises a Clostridial toxin derivative including a native, recombinant Clostridial toxin, recombinant modified toxin, fragments thereof, targeted exocytosis modulators (TEMs), or combinations thereof. In some embodiments, the Clostridial derivative is a botulinum toxin. In alternative embodiments, the Clostridial derivative is a TEM.

In some embodiments, the botulinum neurotoxin can be a modified neurotoxin, that is a botulinum neurotoxin which has at least one of its amino acids deleted, modified or replaced, as compared to a native toxin, or the modified botulinum neurotoxin can be a recombinant produced botulinum neurotoxin or a derivative or fragment thereof. In certain embodiments, the modified toxin has an altered cell targeting capability for a neuronal or non-neuronal cell of interest. This altered capability is achieved by replacing the naturally-occurring targeting domain of a botulinum toxin with a targeting domain showing a selective binding activity for a non- botulinum toxin receptor present in a non- botulinum toxin target cell. Such modifications to a targeting domain result in a modified toxin that is able to selectively bind to a non-botulinum toxin receptor (target receptor) present on a non-botulinum toxin target cell (re-targeted). A modified botulinum toxin with a targeting activity for a non-botulinum toxin target cell can bind to a receptor present on the non-botulinum toxin target cell, translocate into the cytoplasm, and exert its proteolytic effect on the SNARE complex of the target cell. In essence, a botulinum toxin light chain comprising an enzymatic domain is intracellularly delivered to any desired cell by selecting the appropriate targeting domain.

In embodiments, the film comprises a Clostridial toxin as the Clostridial toxin active ingredient. Seven, generally immunologically distinct botulinum neurotoxins have been characterized, these being respectively botulinum neurotoxin serotypes A, B, Ci, D, E, F and G each of which is distinguished by neutralization with type-specific antibodies. The different serotypes of botulinum toxin vary in the animal species that they affect and in the severity and duration of the paralysis they evoke. Botulinum toxin apparently binds with high affinity to cholinergic motor neurons, is translocated into the neuron and blocks the release of acetylcholine.

In aspects of this embodiment, a Clostridial toxin pharmaceutical composition comprises a BoNT/A, a BoNT/B, a BoNT/C₁, a BoNT/D, a BoNT/E, a BoNT/F, a BoNT/G, a TeNT, a BaNT, or a BuNT. In one embodiment, the Clostridial derivative of the present method is a botulinum toxin type A.

In another embodiment, the film comprises a Clostridial toxin variant as the Clostridial toxin active ingredient. In aspects of this embodiment, the film comprises naturally-occurring Clostridial toxin variant or a non-naturally-occurring Clostridial toxin variant. In other aspects of this embodiment, the film comprises a BoNT/A variant, a BoNT/B variant, a BoNT/C₁ variant, a BoNT/D variant, a BοNT/E variant, a BoNT/F variant, a BoNT/G variant, a TeNT variant, a BaNT variant, or a BuNT variant, where the variant is either a naturally-occurring variant or a non-naturally-occurring variant.

The molecular weight of the neurotoxic component of a botulinum toxin complex is about 150 kD. Botulinum toxin is typically made by the *Clostridial botulinum* bacterium as a complex comprising the 150 kD botulinum toxin protein molecule and associated non-toxin proteins. Thus, a botulinum toxin type A complex can be produced by *Clostridial botulinum* bacterium as 900 kD, 500 kD and 300 kD complex forms. The botulinum toxin can further be a recombinant botulinum neurotoxin, such as botulinum toxins produced by *E. coli.*

It is envisioned that any therapeutic amount of Clostridial toxin active ingredient can be included in the film. The therapeutically effective amount of the Clostridial toxin derivative, for example a botulinum toxin, administered according to the present method can vary according to the potency of the toxin and particular characteristics of the condition being treated, including its severity and other various patient variables including size, weight, age, and responsiveness to therapy. The potency of the toxin is expressed as a multiple of the LD₅₀ value for the mouse, one unit (U) of toxin being defined as being the equivalent amount of toxin that kills 50% of a group of 18 to 20 female Swiss-Webster mice, weighing about 20 grams each.

The therapeutically effective amount of the botulinum toxin according to the present method can vary according to the potency of a particular botulinum toxin, as commercially available Botulinum toxin formulations do not have equivalent potency units. For example, one unit of BOTOX^{®} (onabotulinumtoxinA), a botulinum toxin type A available from Allergan, Inc., has a potency unit that is approximately equal to 3 to 5 units of DYSPORT^{®} (abobotulinumtoxinA), also a botulinum toxin type A available from Ipsen Pharmaceuticals. In some embodiments, the amount of abobotulinumtoxinA, (such as DYSPORT^{®}), administered in the present method is about three to four times the amount of onabotulinumtoxinA (such as BOTOX^{®}) administered, as comparative studies have suggested that one unit of onabotulinumtoxinA has a potency that is approximately equal to three to four units of abobotulinumtoxinA MYOBLOC^{®}, a botulinum toxin type B available from Elan, has a much lower potency unit relative to BOTOX^{®}. In some embodiments, the botulinum neurotoxin can be a pure toxin, devoid of complexing proteins, such as XEOMIN^{®} (incobotulinumtoxinA). One unit of incobotulinumtoxinA has potency approximately equivalent to one unit of onabotulinumtoxinA. The quantity of toxin administered and the frequency of its administration will be at the discretion of the physician responsible for the treatment and will be commensurate with questions of safety and the effects produced by a particular toxin formulation. In embodiments, a therapeutically effective amount of the Clostridial toxin derivative may vary depending upon the condition being treated.

In embodiments, a film for administration of a Clostridial toxin active agent comprises about 0.0056 to about 560 ng of a biologically active Clostridial toxin active agent. In embodiments, the film comprises about 0.005 to about 560 ng, about 0.01 to about 560 ng, about 0.1 to about 560 ng, about 1.0 to about 560 ng, about 10 to about 560 ng, about 25 to about 560 ng, about 50 to about 560 ng, about 100 to about 560 ng, about 200 to about 560 ng, about 250 to about 560 ng, about 300 to about 560 ng, about 400 to about 560 ng, about 500 to about 560 ng, about 0.0056 to about 500 ng, about 0.005 to about 500 ng, about 0.01 to about 500 ng, about 0.1 to about 500 ng, about 1.0 to about 500 ng, about 10 to about 500 ng, about 25 to about 500 ng, about 50 to about 500 ng, about 100 to about 500 ng, about 200 to about 500 ng, about 250 to about 500 ng, about 300 to about 500 ng, about 400 to about 500 ng of a Clostridial toxin active. In embodiments, the film comprises at least about 0.0056 ng, at least about 0.005 ng, at least about 0.01 ng, at least about 0.1 ng, at least about 0.5 ng, at least about 1.0 ng, at least about 5 ng, at least about 10 ng, at least about 15 ng, at least about 25 ng, at least about 50 ng, at least about 75 ng, at least about 100 ng, at least about 150 ng, at least about 200 ng, at least about 300 ng, at least about 400 ng, at least about 500 ng, or at least about 560 ng of a biologically active Clostridial toxin active agent. In embodiments, the film comprises at most about 0.0056 ng, at most about 0.005 ng, at most about 0.01 ng, at most about 0.1 ng, at most about 0.5 ng, at most about 1.0 ng, at most about 5 ng, at most about 10 ng, at most about 15 ng, at most about 25 ng, at most about 50 ng, at most about 75 ng, at most about 100 ng, at most about 150 ng, at most about 200 ng, at most about 300 ng, at most about 400 ng, at most about 500 ng, or at most about 560 ng of a biologically active Clostridial toxin active agent. In some embodiments, the film comprises about 0.0056 ng to about 560 ng of a biologically active Clostridial toxin active agent such as BoNT/A per 70 kg of body weight. In some embodiments, the film comprises a toxin load of less than about 10%.

In aspects of this embodiment, the amount of Clostridial toxin active ingredient added to the film formulation is at least about 0.01 U/kg, at least about 0.1 U/kg, at least about 1.0 U/kg, at least about 10 U/kg, at least about 15 U/kg, at least about 25 U/kg, at least about 50 U/kg, at least about 75 U/kg, at least 100 U/kg, at least about 200 U/kg, at least about 300 U/kg, at least about 400 U/kg, at least about 500 U/kg, at least about 600 U/kg, at least about 700 U/kg, at least about 800 U/kg, at least about 900 U/kg, or at least 1000 U/kg. In other aspects of this embodiment, the amount of Clostridial toxin active ingredient added to the film formulation is at most about 0.01 U/kg, at most about 0.1 U/kg, at most about 1.0 U/kg, at most about 10 U/kg, at most about 15 U/kg, at most about 2.5 U/kg, at most about 50 U/kg, at most about 75 U/kg, at most 100 U/kg, at most about 200 U/kg, at most about 300 U/kg, at most about 400 U/kg, at most about 500 U/kg, at most about 600 U/kg, at most about 700 U/kg, at most about 800 U/kg, at most about 900 U/kg, or at most 1000 U/kg. In yet other aspects of this embodiment, the amount of Clostridial toxin active ingredient added to the film formulation is from about 0.01 U/kg to about 1000 U/kg, about 0,1 U/kg to about 1000 U/kg, about 1.0 U/kg to about 1000 U/kg, about 10 U/kg to about 1000 U/kg, about 25 U/kg to about 1000 U/kg, about 50 U/kg to about 1000 U/kg, about 75 U/kg to about 1000 U/kg, about 100 U/kg to about 1000 U/kg, about 200 U/kg to about 1000 U/kg, about 300 U/kg to about 1000 U/kg, about 400 U/kg to about 1000 U/kg, about 500 U/kg to about 1000 U/kg, about 600 U/kg to about 1000 U/kg, about 700 U/kg to about 1000 U/kg, about 800 U/kg to about 1000 U/kg, or about 900 U/kg to about 1000 U/kg. In still other aspects of this embodiment, the amount of Clostridial toxin active ingredient added to the film formulation is from about 0.01 U/kg to about 100 U/kg, about 0.1 U/kg to about 100 U/kg, about 1.0 U/kg to about 100 U/kg. As used herein, the term "unit" or "U" is refers to the LD₅₀ dose, which is defined as the amount of a Clostridial toxin, Clostridial toxin complex or modified Clostridial toxin that killed 50% of the mice injected with the Clostridial toxin, Clostridial toxin complex or modified Clostridial toxin. As used herein, the term "about" when qualifying a value of a stated item, number, percentage, or term refers to a range of plus or minus ten percent of the value of the stated item, percentage, parameter, or term.

It will be appreciated that the film as described above may refer to the formulation used to prepare one film dosage unit. Thus, the film may be the amount of Clostridial toxin active ingredient in a single film dosage unit.

The films as described herein include one or more water-soluble or hydrophilic polymers. A hydrophilic polymer is one that dissolves, disperses, or swells in water or an aqueous solution at room temperature or a body temperature (36.5-37.5 °C). The hydrophilic polymer, in one embodiment, also provides mechanical strength to the film. It will be appreciated that one or more polymers or other ingredients may be used in the film formulation to provide a film having the desired properties including dissolution time, mechanical strength, and/or stability.

The water-soluble polymers, bioadhesive polymers and/or mucosadhesive polymers for use therein may be natural or synthetic. Non-limiting examples of water-soluble polymers include, but are not limited to, polysaccharides, such as, e.g., dextrans (like dextran 1K, dextran 4K, dextran 40K, dextran 60K, and dextran 70K), dextrins, glycogen, inulin, starch or hydroxyalkyl starch derivatives (including hydroxymethyl starch, hydroxyethyl starch, hydroxypropyl starch, hydroxybutyl starch, and hydroxypentyl starch), hetastarch, cellulose and hydroxyalkyl cellulose derivatives (including hydroxymethyl cellulose, hydroxyethyl cellulose (HEC), hydroxypropyl cellulose (HPC), hydroxyethyl methyl cellulose (HEMC), hydroxypropyl methyl cellulose (HPMC), hydroxybutyl cellulose, and hydroxypentyl cellulose), FICOLL, methyl cellulose (MC), carboxymethyl cellulose (CMC); polyvinyl acetates (PVA); polyvinyl pyrrolidones (PVP), also known as povidones, having a K-value of less than or equal to 18, a K-value greater than 18 or less than or equal to 95, or a K-value greater than 95, like PVP 12 (KOLLIDON^{®} 12), PVP 17 (KOLLIDON^{®}17), PVP 25 (KOLLIDON^{®} 25), PVP 30 (KOLLIDON^{®} 30), PVP 90 (KOLLIDON^{®} 90); polyethylene glycols including PEG 100, PEG 200, PEG 300, PEG 400, PEG 500, PEG 600, PEG 700, PEG 800, PEG 900, PEG 1000, PEG 1100, PEG 1200, PEG 1300, PEG 1400, PEG 1500, PEG 1600, PEG 1700, PEG 1800, PEG 1900, PEG 2000, PEG 2100, PEG 2200, PEG 2300, PEG 2400, PEG 2500, PEG 2600, PEG 2700, PEG 2800, PEG 2900, PEG 3000, PEG 3250, PEG 3350, PEG 3500, PEG 3750, PEG 4000, PEG 4250, PEG 4500, PEG 4750, PEG 5000, PEG 5500, PEG 6000, PEG 6500, PEG 75000, PEG 7500, or PEG 8000; and polyethylene imines (PEI); polyethylene oxides (PEO); polypeptides (proteins) including bovine serum albumin, gelatin, and ovalbumin; and polynucleotides such as DNA and RNA. In some embodiments, the polymer is a triblock PEO-PPO-PEO copolymer compound known as a pluronic or poloxamer. In some embodiments, the poloxamer is selected from one or more of Poloxamer 124 (PLURONIC^{®} L44), Poloxamer 181 (PLURONIC^{®} L61), Poloxamer 182 (PLURONIC^{®} L62), Poloxamer 184 (PLURONIC^{®} L64), Poloxamer 188 (PLURONIC^{®} F68), Poloxamer 237 (PLURONIC^{®} F87), Poloxamer 338 (PLURONIC^{®} L108), Poloxamer 407 (PLURONIC^{®} F127).

In some embodiments, the film comprises one or more bioadhesive and/or mucoadhesive polymers. It will be appreciated that one or more of the polymers as used in the film may be bioadhesive/mucoadhesive and water-soluble. The majority of adhesive films are hydrophilic. Where the film is mucoadhesive, the film may undergo swelling to form a chain interaction with the mucin covering the mucosal membrane. Typically, cationic or anionic polymers facilitate adhesion to the mucosal membrane. Non-limiting examples of bioadhesive and/or mucosadhesive polymers include polyacrylic acids; polyacrylates; poly(meth)acrylates; poly(ethylene oxide) (PEO); pectin; sodium alginate; carrageenan; cellulose derivatives including hydroxypropyl cellulose (HPC) and hydroxypropylmethyl cellulose (HPMC), sodium carboxymethyl cellulose (NaCMC); hyaluronic acid (HA); chitosan; alginates, pectin, gelatin, and poloxamers as described above. In embodiments, the film composition comprises one or more cationic and/or anionic polymer.

The film may comprise combinations of water-soluble polymers and/or combinations of bioadhesive and/or mucosadhesive polymers in order to prepare a film having a desired dissolution (rate and/or duration) and/or mechanical properties (e.g. flexibility) and/or adhesion to a mucosal surface. In some embodiments, the film may comprise one or more polymer layers, at least one of which comprises the Clostridial toxin active agent.

In some embodiments, the film compositions comprise about 20 to about 95 w/w% of the at least one polymer. In some embodiments, the film compositions comprise about 20 to about 95 w/w% of the combined water-soluble and/or adhesive polymers. In embodiments, the film composition comprises about 25 to about 95 w/w%, about 30 to about 95 w/w%, about 40 to about 95 w/w%, about 50 to about 95 w/w%, about 60 to about 95 w/w%, about 70 to about 95 w/w%, about 80 to about 95 w/w%, about 90 to about 95 w/w%, about 20 to about 90 w/w%, about 25 to about 90 w/w%, about 30 to about 90 w/w%, about 40 to about 90 w/w%, about 50 to about 90 w/w%, about 60 to about 90 w/w%, about 70 to about 90 w/w%, about 80 to about 90 w/w%, about 20 to about 80 w/w%, about 25 to about 80 w/w%, about 30 to about 80 w/w%, about 40 to about 80 w/w%, about 50 to about 80 w/w%, about 60 to about 80 w/w%, about 70 to about 80 w/w%, about 20 to about 70 w/w%, about 25 to about 70 w/w%, about 30 to about 70 w/w%, about 40 to about 70 w/w%, about 50 to about 70 w/w%, about 60 to about 70 w/w%, about 20 to about 60 w/w%, about 25 to about 60 w/w%, about 30 to about 60 w/w%, about 40 to about 60 w/w%, about 50 to about 60 w/w%, about 20 to about 50 w/w%, about 25 to about 50 w/w%, about 30 to about 50 w/w%, about 40 to about 50 w/w%, about 20 to about 40 w/w%, about 25 to about 40 w/w%, about 30 to about 40 w/w%, about 20 to about 30 w/w%, about 25 to about 30 w/w%, or about 20 to about 25 w/w% of the at least one polymer or the combined polymers. In embodiments, the film composition comprises at least about 20 w/w%, at least about 25 w/w%, at least about 30 w/w%, at least about 40 w/w%, at least about 50 w/w%, at least about 60 w/w%, at least about 70 w/w%, at least about 80 w/w%, at least about 90 w/w%, or at least about 95 w/w% of the at least one polymer or combined polymers. In embodiments, the film composition comprises at most about 20 w/w%, at most about 25 w/w%, at most about 30 w/w%, at most about 40 w/w%, at most about 50 w/w%, at most about 60 w/w%, at most about 70 w/w%, at most about 80 w/w%, at most about 90 w/w%, or at most about 95 w/w% of the at least one polymer or combined polymers.

The Clostridial toxin active ingredient may be uniformly dispersed as a molecular dispersion where the active is dissolved in a polymer matrix formed of one or more polymers described above. In other embodiments, the Clostridial toxin active ingredient is included in the polymer matrix as a particulate dispersion, e.g. suspension in the polymer matrix. In some embodiments, the films as described herein may include at least one mucosal permeation enhancer in order to facilitate permeation of the active agent across or into the mucosal membrane. Suitable mucosal permeation enhancers include, but are not limited to, diethylene glycol monoethyl ether (also known as 2-(2-ethoxyethoxy)ethanol; sold under the trade name TRANSCUTOL^{®}), glyceryl monooleate, dimethyl beta cyclodextrin, saponins, glycolate, sodium taurodihydrofusidate (STDHF), lysophosphatidylcholine (LPC), didecanoyl-L-phosphatidylcholine (DDPC), chitosan, cell penetrating peptides, and surfactants.

Cell penetrating peptides contemplated for use in the film can be hydrophilic, for example due to an abundance of arginine and/or lysine amino acid residues, or amphiphilic, for example due to an abundance of lysine residues. The cell penetrating peptide in one embodiment is a peptide having between about 4-20 amino acid residues, between about 4-18 amino acid residues, between about 4-15 amino acid residues, between about 4-12 amino acid residues, between about 6-20 amino acid residues, between about 6-18 amino acid residues, between about 6-15 amino acid residues, or between about 6-12 amino acid residues. Exemplary hydrophilic cell penetrating peptides include TAT (YGRKKRRQRRR), Antenapedia (RQIKWFQNRRMKWKK), SynB1(RGGRLSYSRRRFSTSTGR), SynB3 (RRLSYSRRRF), PTD-4 (PIRRRKKLRRLK), PTD-5 (RRQRRTSKLMKR) FHV Coat-(35-49) (RRRRNRTRRNRRRVR), BMV Gag-(7-25) (KMTRAQRRAAARRNRWTAR), HTLV-II Rex-(4-16) (TRRQRTRRARRNR), D-Tat (GRKKRRQRRRPPQ), R9-Tat (GRRRRRRRRRPPQ) and pVEC (LLIILRRRIRKQAHAHSK). Exemplary amphiphilic cell penetrating peptides include transportan (GWTLNSAGYLLGKINLKALAALAKKIL), MAP (KLALKLALKLALALKLA), SBP (MGLGLHLLVLAAALQGAWSQPKKKRKV), FBP (GALFLGWLGAAGSTMGAWSQPKKKRKV), MPG (ac-GALFLGFLGAAGSTMGAWSQPKKKRKV-cya), Pep-1 (ac-KETWWETWWTEWSQPKKKRKV-cya), and Pep-2 (ac-KETWFETWFTEWSQPKKKRKV-cya). In one embodiment, the cell penetrating peptide has an amino acid sequence motif that is replicated at least once. For example, RR is an amino acid sequence motif replicated in the TAT cell penetrating peptide.

In some embodiments, the film compositions comprise about 0.0001 to about 15 w/w% of the at least one mucosal permeation enhancer. In embodiments, the film compositions comprise about 0.0001 to about 10 w/w%, about 0.0001 to about 5 w/w%, about 0.0001 to about 1 w/w%, about 0.0001 to about 0.1 w/w%, about 0.0001 to about 0.01 w/w%, about 0.0001 to about 0.001 w/w%, about 0.001 to about 15 w/w%, about 0.001 to about 10 w/w%, about 0.001 to about 5 w/w%, about 0.001 to about 1 w/w%, about 0.001 to about 0.1 w/w%, about 0.001 to about 0.01 w/w%, about 0.01 to about 15 w/w%, about 0.01 to about 10 w/w%, about 0.01 to about 5 w/w%, about 0.01 to about 1 w/w%, about 0.01 to about 0.1 w/w%, about 0.1 to about 15 w/w%, about 0.1 to about 10 w/w%, about 0.1 to about 5 w/w%, about 0.1 to about 1 w/w%, about 1.0 to about 15 w/w%, about 1.0 to about 10 w/w%, about 1.0 to about 5 w/w%, about 5 to about 15 w/w%, about 5 to about 10 w/w%, or about 10 to about 15 w/w%.

In embodiments, the film compositions comprise at least about 0.0001 w/w%, at least about 0.001 w/w%, at least about 0.01 w/w%, at least about 0.1 w/w%, at least about 1.0 w/w%, at least about 5 w/w%, at least about 10 w/w%, or at least about 15 w/w% of a mucosal permeation enhancer. In embodiments, the film compositions comprise at most about 0.0001 w/w%, at most about 0.001 w/w%, at most about 0.01 w/w%, at most about 0.1 w/w%, at most about 1.0 w/w%, at most about 5 w/w%, at most about 10 w/w%, or at most about 15 w/w% of a mucosal permeation enhancer.

In some embodiments, the films as described herein may include at least one dissolution controller in order to facilitate, control, or regulate dissolution of at least one of the water-soluble polymers and thereby facilitate, control, or regulate the rate of dissolution of the film. In some embodiments, the dissolution controller accelerates dissolution of at least one of the water-soluble polymers. In other embodiments, the dissolution controller decreases the rate of dissolution of at least one of the water-soluble polymers. Suitable dissolution controllers include, but are not limited to, sugars, glycerin, propylene glycol and polyvinylpyrrolidones (PVP).

In some embodiments, the film compositions comprise at least about 0.1 to about 15 w/w% of the at least one dissolution controller. In embodiments, the film composition comprises at least about 0.1-10 w/w%, 0.1-5 w/w%, 0.1-1 w/w%, 0.1-0.5 w/w%, 0.5-15 w/w%, 0.5-10 w/w%, 0.5-5 w/w%, 0.5-1 w/w%, 1-15 w/w%, 1-10 w/w%, 1-5 w/w%, 5-15 w/w%, 5-10 w/w%, or 10-15 w/w% of the at least one dissolution controller. In some embodiments, the film composition comprises at least about 0.1 w/w%, at least about 0.5 w/w%, at least about 1 w/w%, at least about 5 w/w%, at least about 10 w/w%, or at least about 15 w/w% of at least one dissolution controller. In some embodiments, the film composition comprises at most about 0.1 w/w%, at most about 0.5 w/w%, at most about 1 w/w%, at most about 5 w/w%, at most about 10 w/w%, or at most about 15 w/w% of at least one dissolution controller.

In some embodiments, the film composition comprises one or more sugars. In embodiments, the one or more sugar is effective to increase dissolution of the film and/or as a stabilizer. As used herein, the term "sugar" refers to a compound comprising one to ten monosaccharide units, e.g., a monosaccharide, a disaccharide, a trisaccharide, and an oligosaccharide comprising four to ten monosaccharide units. Monosaccharides are polyhydroxy aldehydes or polyhydroxy ketones with three or more carbon atoms, including aldoses, dialdoses, aldoketoses, ketoses and diketoses, as well as cyclic forms, deoxy sugars and amino sugars, and their derivatives, provided that the parent monosaccharide has a (potential) carbonyl group. Monosaccharides include trioses, like glyceraldehyde and dihydroxyacetone; tetroses, like erythrose, threose and erythrulose; pentoses, like arabinose, lyxose, ribose, xylose, ribulose, xylulose; hexoses, like allose, altrose, galactose, glucose, gulose, idose, mannose, talose, fructose, psicose, sorbose, tagatose, fucose, rhamnose; heptoses, like sedoheptulose and mannoheptulose; octooses, like octulose and 2-keto-3-deoxy-manno-octonate; nonoses like sialose; and decose. Oligosaccharides are compounds in which at least two monosaccharide units are joined by glycosidic linkages. According to the number of units, they are called disaccharides, trisaccharides, tetrasaccharides, pentasaccharides, hexoaccharides, heptoaccharides, octoaccharides, nonoaccharides, decoaccharides, etc. An oligosaccharide can be unbranched, branched or cyclic. Common disaccharides include, without limitation, sucrose, lactose, maltose, trehalose, cellobiose, gentiobiose, kojibiose, laminaribiose, mannobiose, melibiose, nigerose, rutinose, and xylobiose. Common trisaccharides include, without limitation, raffinose, acarbose, maltotriose, and melezitose.

It is envisioned that any amount of sugar is useful in formulating the Clostridial toxin film compositions disclosed herein, with the proviso that a therapeutically effective amount of the Clostridial toxin active ingredient is recovered from or provided upon administration of a film comprising the amount of sugar. In aspects of this embodiment, the amount of sugar added to the formulation is at least 0.5% (w/v), at least 1.0% (w/v), at least 2.0% (w/v), at least 3.0% (w/v), at least 4.0% (w/v), at least 5.0% (w/v), at least 6.0% (w/v), at least 7.0% (w/v), at least 8.0% (w/v), at least 9.0% (w/v), at least 10% (w/v), at least 15% (w/v), at least 20% (w/v), at least 25% (w/v), at least 30% (w/v), or at least 35% (w/v). In other aspects of this embodiment, the amount of sugar added to the formulation is at most 0.5% (w/v), at most 1.0% (w/v), at most 2.0% (w/v), at most 3.0% (w/v), at most 4.0% (w/v), at most 5.0% (w/v), at most 6.0% (w/v), at most 7.0% (w/v), at most 8.0% (w/v), at most 9.0% (w/v), at most 10% (w/v), at most 15% (w/v), at most 20% (w/v), at most 25% (w/v), at most 30% (w/v), or at most 35% (w/v).

Aspects of the present pharmaceutical compositions provide, in part, one or more polyols. In embodiments, the one or more polyol is effective to increase dissolution of the film and/or as a stabilizer. As used herein, the term "polyol" is synonymous with "sugar alcohol," "polyhydric alcohol," and "polyalcohol" and refers to a sugar derivative having an alcohol group (CH₂OH) instead of the aldehyde group (CHO), such as, e.g., mannitol from mannose, xylitol from xylose, and lactitol from lactulose. It is envisioned that any polyol is useful in formulating a Clostridial toxin film compositions disclosed in the present specification, with the proviso that a therapeutically effective amount of the Clostridial toxin active ingredient is recovered using this polyol. Non-limiting examples of polyols include, glycol, glycerol, arabitol, erythritol, xylitol, maltitol, sorbitol (gluctiol), mannitol, inositol, lactitol, galactitol (iditol), isomalt. Other non-limiting examples of sugar excipients can be found in, e.g., Ansel (1999); Gennaro (2000); Hardman; and Rowe (2003), each of which is hereby incorporated by reference in its entirety.

It is envisioned that any amount of polyol is useful in formulating a Clostridial toxin film composition disclosed in the present specification, with the proviso that a therapeutically effective amount of the Clostridial toxin active ingredient is recovered using this polyol amount. In aspects of this embodiment, the amount of polyol added to the formulation is at least 0.5% (w/v), at least 1.0% (w/v), at least 2.0% (w/v), at least 3.0% (w/v), at least 4.0% (w/v), at least 5.0% (w/v), at least 6.0% (w/v), at least 7.0% (w/v), at least 8.0% (w/v), at least 9.0% (w/v), at least 10% (w/v), at least 15% (w/v), at least 20% (w/v), at least 25% (w/v), at least 30% (w/v), or at least 35% (w/v). In other aspects of this embodiment, the amount of polyol added to the formulation is at most 0.5% (w/v), at most 1.0% (w/v), at most 2.0% (w/v), at most 3.0% (w/v), at most 4.0% (w/v), at most 5.0% (w/v), at most 6.0% (w/v), at most 7.0% (w/v), at most 8.0% (w/v), at most 9.0% (w/v), at most 10% (w/v), at most 15% (w/v), at most 20% (w/v), at most 25% (w/v), at most 30% (w/v), or at most 35% (w/v).

The films as described herein may further comprise one or more excipients including, but not limited to film agents, stabilizers, plasticizers, antioxidants, penetration enhancers, charge adjusting agents, surfactants, charge modifiers, preservatives, buffers, salts, osmolality- or osmolarity adjusting agents, emulsifying agents, and/or sweetening or flavoring agents.

In some embodiments, the film composition comprises one or more surfactants. The surfactant may be a natural or synthetic amphiphilic compound. Suitable surfactants can be nonionic, zwitterionic, or ionic. Non-limiting examples of surfactants include polysorbates like polysorbate 20 (TWEEN^{®} 20), polysorbate 40 (TWEEN^{®} 40), polysorbate 60 (TWEEN^{®} 60), polysorbate 61 (TWEEN^{®} (1), polysorbate 65 (TWEEN^{®} 65), polysorbate 80 (TWEEN^{®} 80), and polysorbate 81 (TWEEN^{®} 81); poloxamers (polyethylene-polypropylene triblock copolymers), like poloxamer 124 (PLURONIC^{®} L44), poloxamer 181 (PLURONIC^{®} L61), poloxamer 182 (PLURONIC^{®} L(2), poloxamer 184 (PLURONIC^{®} L64), poloxamer 188 (PLURONIC^{®} F68), poloxamer 237 (PLURONIC^{®} F87), poloxamer 338 (PLURONIC^{®} L108), poloxamer 407 (PLURONIC^{®} F127); polyoxyethyleneglycol dodecyl ethers, like BRIJ^{®} 30, and BRIJ^{®} 35; 2-dodecoxyethanol (LUBROL^{®}-PX); polyoxyethylene octyl phenyl ether (TRITON^{®} X-100); sodium dodecyl sulfate (SDS); 3-[(3-Cholamidopropyl)dimethylammonio]-1-propanesulfonate (CHAPS); 3-[(3-Cholamidopropyl)dimethylammonio]-2-hydroxy-1-propanesulfonate (CHAPSO); sucrose monolaurate; sodium cholate; and benzalkonium chloride.

It is envisioned that any amount of surfactant is useful in formulating a Clostridial toxin film composition, with the proviso that a therapeutically effective amount of the Clostridial toxin active ingredient is recovered from or provided upon administration of a film comprising the surfactant amount. In aspects of this embodiment, the film compositions comprise about 0.1-35 % of one or more surfactants. In embodiments, the film composition comprises about 0.1-30%, 0.1-30%, 0.1-25%, 0.1-20%, 0.1-15%, 0.1-10%, 0.1-5 %, 0.1-4%, 0.1-3%, 0.1-2%, 0.1-1%, 0.1-0.5%, 0.5-35%, 0.5-30%, 0.5-30%, 0.5-25%, 0.5-20%, 0.5-15%, 0.5-10%, 0.5-5 %, 0.5-4%, 0.5-3%, 0.5-2%, 0.5-1%, 1-35%, 1-30%, 1-30%, 1-25%, 1-20%, 1-15%, 1-10%, 1-5 %, 1-4%, 1-3%, 1-2%, 2-35%, 2-30%, 2-30%, 2-25%, 2-20%, 2-15%, 2-10%, 2-5 %, 2-4%, 2-3%, 3-35%, 3-30%, 3-30%, 3-25%, 3-20%, 3-15%, 3-10%, 3-5 %, 3-4%, 4-35%, 4-30%, 4-30%, 4-25%, 4-20%, 4-15%, 4-10%, 5-35%, 5-30%, 5-30%, 5-25%, 5-20%, 5-15%, 5-10%, 10-35%, 10-30%, 10-30%, 10-25%, 10-20%, 10-15%, 15-35%, 15-30%, 15-30%, 15-25%, 15-20%, 20-35%, 20-30%, 20-30%, 20-25%, 25-35%, 25-30%, or 30-35% of one or more surfactants. The % amount can refer to either of w/w% or % w/v.

In some embodiments, the amount of surfactant added to the formulation is at least about 0.1% (w/v), at least about 0.5% (w/v), at least 1.0% (w/v), at least 2.0% (w/v), at least 3.0% (w/v), at least 4.0% (w/v), at least 5.0% (w/v), at least 6.0% (w/v), at least 7.0% (w/v), at least 8.0% (w/v), at least 9.0% (w/v), at least 10% (w/v), at least 15% (w/v), at least 20% (w/v), at least 25% (w/v), at least 30% (w/v), or at least 35% (w/v). In other aspects of this embodiment, the amount of surfactant added to the formulation is at most 0.5% (w/v), at most 1.0% (w/v), at most 2.0% (w/v), at most 3.0% (w/v), at most 4.0% (w/v), at most 5.0% (w/v), at most 6.0% (w/v), at most 7.0% (w/v), at most 8.0% (w/v), at most 9.0% (w/v), at most 10% (w/v), at most 15% (w/v), at most 20% (w/v), at most 25% (w/v), at most 30% (w/v), or at most 35% (w/v).

In yet other aspects of this embodiment, the amount of surfactant added to the formulation is at least 0.5% (v/v), at least 1.0% (v/v), at least 2.0% (v/v), at least 3.0% (v/v), at least 4.0% (v/v), at least 5.0% (v/v), at least 6.0% (v/v), at least 7.0% (v/v), at least 8.0% (v/v), at least 9.0% (v/v), at least 10% (v/v), at least 15% (v/v), at least 20% (v/v), at least 25% (v/v), at least 30% (v/v), or at least 35% (v/v). In other aspects of this embodiment, the amount of surfactant added to the formulation is at most 0.5% (v/v), at most 1.0% (v/v), at most 2.0% (v/v), at most 3.0% (v/v), at most 4.0% (v/v), at most 5.0% (v/v), at most 6.0% (v/v), at most 7.0% (v/v), at most 8.0% (v/v), at most 9.0% (v/v), at most 10% (v/v), at most 15% (v/v), at most 20% (v/v), at most 25% (v/v), at most 30% (v/v), or at most 35% (v/v).

In some embodiments, the film composition comprises one or more stabilizers. Exemplary stabilizers include, but are not limited to, surfactants, polymers, polyols, a poloxamer, albumin, gelatin, trehalose, proteins, sugars, polyvinylpyrrolidone, N-acetyl-tryptophan ("NAT"), caprylate (i.e. sodium caprylate), a polysorbate (*i.e.* P80), amino acids, and divalent metal cations such as zinc. In some embodiments, one or more of the stabilizers are animal-free stabilizers as described in U.S. published application no. 2012/0207787, which is incorporated herein by reference.

It is envisioned that any amount of stabilizer is useful in formulating a Clostridial toxin film compositions, with the proviso that a therapeutically effective amount of the Clostridial toxin active ingredient is recovered from or delivered upon administration of a film using this stabilizer amount. In aspects of this embodiment, the amount of surfactant added to the formulation about 1.0-10% (w/v). In embodiments, the amount of surfactant added to the formulation is about 1.0-10% (w/v), 2.0-10% (w/v), 3.0-10% (w/v), 4.0-10.0% (w/v), 5.0-10% (w/v), 6.0-10.0% (w/v), 7.0-10.0% (w/v), 8.0-10.0% (w/v), or 9.0-10,0% (w/v).

In some embodiments, the amount of stabilizer is at least 1.0% (w/v), at least 2.0% (w/v), at least 3.0% (w/v), at least 4.0% (w/v), at least 5.0% (w/v), at least 6.0% (w/v), at least 7.0% (w/v), at least 8.0% (w/v), at least 9.0% (w/v), or at least 10% (w/v). In other aspects of this embodiment, the amount of stabilizer added to the formulation is at most 1.0% (w/v), at most 2.0% (w/v), at most 3.0% (w/v), at most 4.0% (w/v), at most 5.0% (w/v), at most 6.0% (w/v), at most 7.0% (w/v), at most 8.0% (w/v), at most 9.0% (w/v), or at most 10% (w/v).

In embodiments, the film compositions comprise one or more plasticizers in order to improve the physical properties of the film and/or to alter the dissolution kinetics of the film. In some embodiments, a plasticizer may be used to adjust the physical properties of the film including without limitation soften the film, increase the durability of the film, or increase the film flexibility. Suitable plasticizers as known in the art may be used in the film compositions herein. In some non-limiting embodiments, the plasticizer is selected from glycerin, polyethylene glycol (PEG) as described further above, propylene glycol (PG), alcohols, and polyols.

In embodiments, the amount of plasticizer added to the formulation is about 1.0-10% (w/v), 2.0-10% (w/v), 3.0-10% (w/v), 4.0-10.0% (w/v), 5.0-10% (w/v), 6.0-10.0% (w/v), 7.0-10.0% (w/v), 8.0-10.0% (w/v), or 9.0-10.0% (w/v). In some embodiments, the amount of plasticizer is at least 1.0% (w/v), at least 2.0% (w/v), at least 3.0% (w/v), at least 4.0% (w/v), at least 5.0% (w/v), at least 6.0% (w/v), at least 7.0% (w/v), at least 8.0% (w/v), at least 9.0% (w/v), or at least 10% (w/v). In other aspects of this embodiment, the amount of plasticizer added to the formulation is at most 1.0% (w/v), at most 2.0% (w/v), at most 3.0% (w/v), at most 4.0% (w/v), at most 5.0% (w/v), at most 6.0% (w/v), at most 7.0% (w/v), at most 8.0% (w/v), at most 9.0% (w/v), or at most 10% (w/v).

In some embodiments, the film composition comprises one or more charge modifying agents. Exemplary charge modifying agents include, but are not limited to, benzalkonium chloride, sodium chloride, mannitol, glycerin.

In embodiments, the amount of charge modifying agent added to the formulation is about 0.01-1.0% (w/v), 0.05-1.0% (w/v), 0.1-1.0% (w/v), 0.2-1.% (w/v), 0.3-1.0% (w/v), 0.4-1.0% (w/v), 0.5-1.0% (w/v), 0.6-1.0% (w/v), 0.6-1.0% (w/v), 0.7-1.0% (w/v), 0.8-1.0% (w/v), or 0.9-1.0% (w/v). In some embodiments, the amount of charge modifying agent is at least 0.01% (w/v), at least 0.05% (w/v), at least 0.1% (w/v), at least 0.2% (w/v), at least 0.3% (w/v), at least 0.4% (w/v), at least 0.5% (w/v), at least 0.6% (w/v), at least 0.7% (w/v), at least 0.8% (w/v), at least 0.9% (w/v), or at least 1.0% (w/v). In other aspects of this embodiment, the amount of the amount of charge modifying agent is at most 0.01% (w/v), at most 0.05% (w/v), at most 0.1% (w/v), at most 0.2% (w/v), at most 0.3% (w/v), at most 0.4% (w/v), at most 0.5% (w/v), at most 0.6% (w/v), at most 0.7% (w/v), at most 0.8% (w/v), at most 0.9% (w/v), or at most 1.0% (w/v).

Various buffers and means for adjusting pH can be used to prepare a film composition disclosed herein, provided that the resulting preparation is pharmaceutically acceptable. Such buffers include, without limitation, acetate buffers, citrate buffers, phosphate buffers, neutral buffered saline, phosphate buffered saline and borate buffers. It is understood that acids or bases can be used to adjust the pH of a pharmaceutical composition as needed. It is envisioned that any buffered pH level can be useful in formulating a Clostridial toxin film composition. In an aspect of this embodiment, an effective pH level is at least about pH 5.0, at least about pH 5.5, at least about pH 6.0, at least about pH 6.5, at least about pH 7.0 or at about pH 7.5. In another aspect of this embodiment, an effective pH level is at most about pH 5.0, at most about pH 5.5, at most about pH 6.0, at most about pH 6.5, at most about pH 7.0 or at most about pH 7.5. In yet another aspect of this embodiment, an effective pH level is about pH 5.0 to about pH 8.0, an effective pH level is about pH 5.0 to about pH 7.0, an effective pH level is about pH 5.0 to about pH 6.0, is about pH 5.5 to about pH 8.0, an effective pH level is about pH 5.5 to about pH 7.0, an effective pH level is about pH 5.5 to about pH 5.0, is about pH 5.5 to about pH 7.5, an effective pH level is about pH 5.5 to about pH 6.5. It will be appreciated that the pH of the film may be adjusted or selected based on the administration site. For example, the pH of the nasal cavity is about 5.0-6.5 for adults. Thus, the pH of a film for nasal administration should be similar to the nasal cavity in order to reduce or prevent irritation at the nasal site, prevent differences in nasal absorption due to ionization of the active agent or excipients, and prevent growth of pathogenic bacteria in the nasal cavity. In other embodiments, the pH of a film, including a film for nasal delivery, can have a pH of about 3 to about 10.

In embodiments, the films described herein include one or more antioxidants. Pharmaceutically acceptable antioxidants include, without limitation, sodium metabisulfite, sodium thiosulfate, sodium bisulfite, acetylcysteine, butylated hydroxyanisole and butylated hydroxy toluene, ascorbic acid and derivatives, and tocopherol. Useful preservatives include, without limitation, benzalkonium chloride, phenyl ethyl alcohol, benzoyl alcohol, ethylene diaminetetraacetic acid (EDTA), parabens, chlorobutanol, thimerosal, phenylmercuric acetate, phenylmercuric nitrate, a stabilized oxy chloro composition, such as, e.g., PURITE^{®} and chelants, such as, e.g., DTPA or DTPA-bisamide, calcium DTPA, and CaNaDTPA-bisamide. Preferably, antioxidants for use in the films do not affect absorption of the active agent and/or do not cause nasal irritation for nasal films.

In embodiments, the film compositions comprise about 0.1-2% w/w of at least one antioxidant. In embodiments, the film compositions comprise about 0.1-1.5% w/w, 0.1-1% w/w, 0.1-0.75% w/w, 0.1-0.5% w/w, 0.5-2% w/w, 0.5-1.5% w/w, 0.5-1% w/w, 0.5-0.75% w/w, 0.75-1.5% w/w, 0.75-1% w/w, 1-2% w/w, 0.1-1.5% w/w or 1.5-2% w/w of the at least one antioxidant. In embodiments, the film composition comprises at least about 0.1% w/w, 0.5% w/w, 0.75% w/w, 1% w/w, 1.5% w/w, or 2% w/w of the at least one antioxidant. In other embodiments, the film composition comprises not more than 0.1% w/w, 0.5% w/w, 0.75% w/w, 1% w/w, 1.5% w/w, or 2% w/w of the at least one antioxidant.

In embodiments, the films described herein include one or more charge adjusting agents or adjustors. Charge adjustors useful in a pharmaceutical composition include, without limitation, salts such as, e.g., benzalkonium chloride, sodium chloride, and potassium chloride.

In embodiments, the film compositions comprise about 0.01-1% w/w of at least one charge adjusting agent or adjustors. In embodiments, the film compositions comprise about 0.01-0.5% w/w, 0.01-0.75% w/w, 0.1-1% w/w, 0.1-0.5% w/w, 0.1-0.75% w/w, 0.5-1% w/w, 0.5-0.75% w/w, or 0.75-1% of the at least one charge adjusting agent or adjustor. In embodiments, the film composition comprises at least about 0.01% w/w, 0.1% w/w, 0.5% w/w, 0.75% w/w, or 1% w/w of the at least one charge adjusting agent or adjustor. In embodiments, the film composition comprises not more than about 0.01% w/w, 0.1% w/w, 0.5% w/w, 0.75% w/w, or 1% w/w of the at least one charge adjusting agent or adjuster.

In embodiments, the film compositions comprise one or more preservative agents. Exemplary preservative agents include, but are not limited to benzyl alcohol, benzoic acid, phenol, parabens and sorbic acid.

Pharmaceutical compositions can further include one or more excipients, including but not limited to, surface active agents; dispersing agents; inert diluents; granulating and disintegrating agents; binding agents; lubricating agents; physiologically degradable compositions such as gelatin; aqueous vehicles and solvents; oily vehicles and solvents; suspending agents; dispersing or wetting agents; emulsifying agents, demulcents; buffers; salts; thickening agents; fillers, and other ingredients known in the art and described, for example in Genaro, ed., 1985, Remington's Pharmaceutical Sciences, Mack Publishing Co., Easton, Pa., which is incorporated herein by reference. It is understood that these and other substances known in the art of pharmacology can be included in the films described herein.

In embodiments, a plurality of excipients may be included in the film composition. In embodiments, the film composition comprises, e.g., at least two excipients, at least three excipients, at least four excipients, at least five excipients, at least six excipients, at least seven excipients, at least eight excipients, or more. In other aspects of this embodiment, a Clostridial toxin film composition comprises, e.g., at most two excipients, at most three excipients, at most four excipients, at most five excipients, at most six excipients, at most seven excipients or at most eight excipients. In other aspects of this embodiment, a Clostridial toxin film composition can comprise, e.g., about 2-10 excipients, about 2-8 excipients, about 2-6 excipients, about 2-4 excipients, about 3-10 excipients, about 3-8 excipients, about 3-6 excipients, about 3-4 excipients, about 4-10 excipients, about 4-8 excipients, or about 4-6 excipients.

In some embodiments, the film comprises a Clostridial toxin active ingredient, an anionic bioadhesive/mucosadhesive polymer, a hydrophilic bioadhesive/mucosadhesive polymer (which may additionally act as a stabilizer for the active agent), a plasticizer, a penetration enhancer, a dissolution controller, and, an optional surfactant. In some embodiments, the film comprises a Clostridial toxin active ingredient, a water-soluble polymer that dissolves in water, a mucoadhesive polymer, a plasticizer, a penetration enhancer, a dissolution controller, and a surfactant. In some embodiments, the film composition comprises a Clostridial toxin active ingredient, a water-soluble polymer that dissolves in water, a mucoadhesive polymer, a plasticizer, an antioxidant, and a penetration enhancer. In some embodiments, the film composition comprises a Clostridial toxin active ingredient, a water-soluble polymer that dissolves in water, a mucoadhesive polymer, a plasticizer, a stabilizer, a penetration modifier, and a surfactant. In some embodiments, the film composition comprises a Clostridial toxin active ingredient, a mucoadhesive polymer, a plasticizer, a penetration enhancer, a dissolution controller, a charge adjusting agent, and at least two surfactants. In some embodiments, the film composition comprises a Clostridial toxin active ingredient, a water-soluble polymer that dissolves in water, a mucoadhesive polymer, a stabilizer, and at least two penetration modifiers. In some embodiments, the film composition comprises a Clostridial toxin active ingredient, two or more water-soluble polymers that dissolve in water, a plasticizer, a dissolution controller, and a surfactant. In some embodiments, the film comprises a Clostridial toxin active ingredient, at least two water-soluble polymers that dissolve in water, a plasticizer, a stabilizer, at least two penetration modifiers, a dissolution controller, a charge adjusting agent, and a surfactant. In some embodiments, the film comprises a Clostridial toxin active ingredient, at least two mucoadhesive polymers (where at least one polymer may additionally act as a stabilizer for the active agent), a plasticizer, an antioxidant, at least two penetration modifiers or enhancers, and a surfactant. In embodiments, the film comprises a Clostridial toxin active ingredient, at least two water-soluble polymers that dissolve in water, a mucoadhesive polymer, a plasticizer, an antioxidant, a stabilizer, a penetration enhancer or modifier, a dissolution controller, and at least two surfactants.

The water-soluble polymer in the dissolving film dissolves in water and/or in physiologic fluid at body temperature (36.5-37.5 °C). The film comprises a sufficient amount of the water-soluble polymer, in one embodiment, so that release of the active agent is controlled by the rate at which the water-soluble polymer dissolves. It will be appreciated that the dissolvable or erodible film described herein intends a film where the water-soluble polymer component dissolves rather than degrades where the polymer breaks by chemical reaction into smaller molecular weight oligomers or polymers. In one embodiment, substantially all of the dissolving film when placed in contact with a physiologic fluid or water at 36.5-37.5 °C dissolves in a time period of between 10 seconds to 10 minutes. "Substantially all of the dissolving film" dissolves refers to more than about 60%, more than about 75%, or more than about 80%, 85% or 90% or 95% of the film has dissolved in the time period. In one embodiment, the time period is between about 10 seconds to 10 minutes, and in other embodiments, the time period is between about 10 seconds to 8 minutes, between about 10 seconds to 7 minutes, between about 10 seconds to 6 minutes, between about 10 seconds to 5 minutes, between about 10 seconds to 4 minutes, between about 10 seconds to 3 minutes, between about 10 seconds to 2 minutes, between about 10 seconds to 1 minute, between about 10 seconds to 50 seconds, between about 10 seconds to 40 seconds, between about 10 seconds to 30 seconds, between about 20 seconds to 10 minutes, between about 20 seconds to 8 minutes, between about 20 seconds to 7 minutes, between about 20 seconds to 6 minutes, between about 20 seconds to 5 minutes, between about 20 seconds to 4 minutes, between about 20 seconds to 3 minutes, between about 20 seconds to 2 minutes, between about 20 seconds to 1 minute, between about 20 seconds to 50 seconds, between about 20 seconds to 40 seconds, between about 20 seconds to 30 seconds, between about 30 seconds to 10 minutes, between about 30 seconds to 8 minutes, between about 30 seconds to 7 minutes, between about 30 seconds to 6 minutes, between about 30 seconds to 5 minutes, between about 30 seconds to 4 minutes, between about 30 seconds to 3 minutes, between about 30 seconds to 2 minutes, between about 30 seconds to 1 minute, or between about 30 seconds to 50 seconds.

In one embodiment, the dissolving film has a uniform thickness across its length and width dimensions. In some embodiments, the dissolving film has a thickness of about 1 µm to about 5 mm, about 10 µm to about 2 mm, about 20 µm to about 1 mm, about 50 µm to about 0.5 mm, about 100 µm to about 250 µm, or about 150 µm. In some embodiments, the dissolving film is in a size that can comfortably be placed in a nasal passageway or nasal cavity of a human subject. For example, in some embodiments, the film may have an area of about 0.1 cm² to about 5 cm², preferably about 0.5 cm² to about 3 cm², more preferably about 0.5 cm² to about 2 cm².

The films may provide rapid dissolution of the film and/or improved delivery of the Clostridial toxin active ingredient in order to prevent migration of the active away from the administration site. Without being limited by theory, in embodiments polymers and/or dissolution controllers with positive charges may neutralize the negative charge of the Clostridial toxin active ingredient in order to enhance mucosal delivery of the active. In embodiments, the polymer may create supersaturation conditions at the application site in order to accelerate the effect of a permeation enhancer.

In some embodiments, the films further include a non-dissolvable or erodible backing layer to protect the polymer film layer and/or to ease handling. In some embodiments, the backing layer is a releasable backing that is removed before or after placing the polymer film at the administration site. The backing layer may be formed of any suitable material as known in the art including, but not limited to, polyesters, polyurethanes, polyethylenes (e.g. polyethylene terepthalate), polypropylenes, and metal foils such as an aluminum foil. In some embodiments, the polymer film is cast or extruded directly onto the backing member.

The films as described herein may be manufactured using any suitable method as known in the art. In embodiments, the films are prepared using a solvent casting method. Briefly, the Clostridial toxin active ingredient is mixed with the one or more polymers, the selected excipients, and a suitable solvent to form a solution or suspension. Suitable solvents are known in the art and may be chosen to ensure the components of the film composition (e.g. polymers) are sufficiently soluble. In some exemplary embodiments, the solvent is an aqueous solvent or water-based solvent, an alcohol, or an organic solvent. In some embodiments, the aqueous solvent is at least 25-75% water. In other embodiments, the solvent is water. In some non-limiting embodiments, the alcohol solvent is selected from ethyl alcohol, propyl alcohol, butyl alcohol, and amyl alcohol. The solution or suspension is cast onto a suitable substrate at the desired thickness. The film may be any suitable thickness as required to include the active agent and components and being for nasal administration. In some embodiment, the film has a thickness of about 50-150 µm. The cast solution or suspension is dried using suitable techniques as known in the art taking care not to affect the Clostridial toxin active ingredient. The dried film is cut to a suitable size and shape to provide the desired dosage of the Clostridial toxin active ingredient and/or provide a suitably sized dosage form for administration at a desired site. In other embodiments, the films are prepared by hot melt extrusion methods as known in the art where the ingredients are melted into a film taking care not to affect the Clostridial toxin active ingredient. In this method, the polymers and excipients should be chosen such that the melting temperature is at or below a temperature that would denature or otherwise adversely affect the Clostridial toxin active ingredient. In other embodiments, the film may be formed with a printing technology.

Some exemplary composition components for forming films as described herein are set forth in Table 1.

**Table 1: Film Compositions**

| | Range |
|---|---|
| Clostridial toxin active ingredient | 0.0056-560 ng/film |
| Water soluble/ dissolving polymer | 0-95% w/w |
| Adhesive polymer | 0-95% w/w |
| Stabilizer | 1-10% w/w |
| Plasticizer (optional) | 1-10% w/w |
| Antioxidant | 0.1-5% w/w |
| Penetration enhancer (optional) | 0.0001-15% w/w |
| Dissolution controller | 0.1-15% w/w |
| Charge adjusting agent (optional) | 0.01-1% w/w |
| Surfactant (optional) | 0.1-10% w/w |
| Solvent | As needed |

It will be appreciated that each of the films includes at least one of a water soluble/ dissolving polymer and an adhesive polymer. In some non-limiting embodiments, the formulations for preparing the film comprise about 50-95 % w/w of the polymer or combined polymers.

It will be appreciated that the films may be textured during or after formation in order to enhance delivery of the Clostridial toxin active ingredient through microabrasion. In some embodiments, the film may be cast or extruded such that the administration surface (surface to be placed in contact the administration site) includes one or more microstructures to disrupt the membrane or skin at the administration site and aid transport of the active ingredient across the membrane/skin. In other embodiments, the film may be textured after formation by methods as known in the art.

### III. Methods of Treatment

The films as described herein are suitable for use in any environment that can provide sufficient moisture to dissolve the film and provide administration of the active agent. Suitable administration sites include, but are not limited to the oral cavity (e.g. buccal and sublingual), the vagina, the urethra, the eye, the gastrointestinal tract, the rectum, and the nose or nasal cavities. Typically, the administration provides a local effect (local administration).

In one embodiment, the film is suitable for nasal administration of a Clostridial toxin active ingredient. Preferably, nasal administration of the film provides for a local effect of the Clostridial toxin active ingredient. Topical administration of a botulinum neurotoxin to the nasal cavity by wiping the formulation in the nasal cavity has been described in U.S. Publication No. 2014/0120077. However, this earlier formulation may diffuse to areas other than the desired site. Further, the amount of this formulation is not specifically defined and excess formulation must be removed after a specified dwell time. The present film provides localized administration as the film formulation is administered and adhered to a specific site within the nasal cavity. Further, adherence of the film to the administration site provides an improved safety as the formulation is administered to a specific site.

In some embodiments, the film is suitable for treating rhinitis or the symptoms associated with rhinitis. It will be appreciated that rhinitis as used herein applies to allergic rhinitis such as seasonal allergic rhinitis, non-allergic or vasomotor rhinitis, and infectious rhinitis due to viral or bacterial infection. Non-allergic rhinitis may be caused by chemicals, smoking, hormones, and medication. In some embodiments, the film is suitable for treating one or more symptoms of rhinitis including, but not limited to, nasal inflammation, nasal congestion, rhinorrhea, sneezing, and itching. In some embodiments, the film is suitable for treating one or more symptoms including, but not limited to, nasal inflammation, nasal congestion, rhinorrhea, sneezing, and itching, where the symptom is not associated with rhinitis. In embodiments, the films described herein can be useful for the treatment, reduction of symptoms and/or prevention of, for example, allergic rhinitis, nasal congestion, post nasal drip, sneezing, cough, sleep apnea, and snoring.

The film may be administered to the mucous membrane of the nasal cavity. Preferably, the film has sufficient bioadhesion to securely adhere to the mucous membrane at the administration site. It will be appreciated that the film may be applied to any portion of the nasal cavity as necessary to relieve the symptoms or disorder being treated. In embodiments, where rhinitis is being treated, the film may be administered to at least one of the inferior turbinate, middle turbinate and/or the superior turbinate. It will be appreciated that at least one or more than one film may be applied to the nasal cavity at a particular time. In some embodiments, one film may be applied to one area of the nasal cavity while one or more films are applied to a different area of the nasal cavity in order to treat or diminish the different symptoms of the disorder being treated. It will further be appreciated that a film maybe applied via one or both nostrils at a time. The film may be applied manually or using a suitable applicator. For administration, the film is contacted with the mucosal membrane and may be pressed against the mucosa until the film adheres to the administration site. In order to ensure adequate and/or rapid dissolution of the film, saline or water drops may be applied to the nasal cavity before or after the film is applied.

### IV. Examples

The following examples are put forth so as to provide those of ordinary skill in the art with a complete disclosure and description of how to manufacture the nasal films of the invention, and are not intended to limit the scope of that which the inventors regard as the invention. Efforts have been made to ensure accuracy with respect to numbers (e.g., amounts, temperatures, etc.) but some errors and deviations should be accounted for. Unless indicated otherwise, parts are parts by weight, temperature is in degrees Celsius (° C), and pressure is at or near atmospheric.

### EXAMPLE 1

### Film Compositions

Film compositions are prepared by adding the respective component in the indicated amount to a suitable solvent.

**Table 2: Film Compositions**

| Component | Formulation | | | | | | | | |
|---|---|---|---|---|---|---|---|---|---|
| | 1 | 2 | 3 | 4 | 5 | 6 | 7 | 8 | 9 |
| Clostridial toxin active ingredient | 0.0056-560 ng/film | | | | | | | | |
| Hyaluronic acid (% w/w) | 50-95 | | | | | 1-10 | 0-10 | | 50-90 |
| polyethylene oxide (% w/w) | | 50-95 | | | 50-80 | 50-95 | | | |
| Gelatin (% w/w) | | | 50-95 | | | | 50-95 | | 0.01-10 |
| Hydroxypropyl cellulose (% w/w) | | 1-30 | | 20-80 | | | | 50-95 | |
| PEG-PPG-PEG triblock copolymer (poloxamer 407) (% w/w) | 2-10 | | 1-5 | | 5-50 | | | 1-10 | 1-10 |
| Glycerin (% w/w) | 1-10 | | | 1-10 | | 1-10 | | 1-10 | |
| Polyethylene glycol (PEG) (% w/w) | | 1-10 | 1-10 | | | | 1-10 | | 1-10 |
| BHA (butylated hydroxyanisole) (% w/w) | | 0.1-2 | | | | | | 0.1-2 | 0.1-2 |
| Trehalose | | | 1-10 | | 1-10 | | 1-10 | | 1-10 |
| diethylene glycol monoethyl ether (TRANSCUTOL^{®}) (% w/w) | | 0-10 | | 0.1-10 | | | | 0.1-10 | |
| glyceryl monooleate (% w/w) | 1-5 | | | | 1-5 | | 1-5 | 1-5 | |
| cell-penetrating peptide (% w/w) | | | 0.1-10 | | 0.1-10 | | 0.1-10 | | 0-5 |
| Sucrose (% w/w) | 1-10 | | | | | 1-10 | | | 1-10 |
| Silicon dioxide (% w/w) | 1-10 | | | 1-10 | | | 1-10 | | |
| benzalkonium chloride (BAK) (% w/w) | | | | 0.01-1 | | | 0.01-1 | | |
| Polyoxyethylene (20) sorbitan monooleate (POLYSORBATE^{®} 80) (% w/w) | 0.1-5 | | 0.1-10 | 0.1-10 | | | 0.1-5 | 0.1-5 | 0.1-5 |
| Sorbitan monolaurate (SPAN^{®} 20) (% w/w) | | | | 0.1-10 | | 0.1-10 | | | 0.1-5 |
| Solvent | q.s. | q.s. | q.s. | q.s. | q.s. | q.s. | q.s. | q.s. | q.s. |

Embodiments:
1. A method of treating rhinitis or a symptom of rhinitis, comprising:
   administering a biodegradable or biocompatible film to a nasal cavity of a subject thereby relieving one or more symptoms of rhinitis, the film comprising:
   a therapeutically effective amount of a Clostridial toxin active ingredient;
   at least one polymer selected from a hydrophilic polymer and a bioadhesive/mucoadhesive polymer; and
   at least one of:
      (a) at least one mucosal permeation enhancer; and
      (b) at least one dissolution controller.
2. The method of embodiment 1, wherein the film comprises at least about 0.0056-560 ng of the Clostridial toxin active ingredient.
3. The method of the separate or combined embodiments 1-2, wherein the film dissolves at the administration site in less than about 10 minutes.
4. The method of the separate or combined embodiments 1-3, wherein the film dissolves at the administration site in about 10 seconds to about 10 minutes.
5. The method of the separate or combined embodiments 1-4, wherein the Clostridial toxin active ingredient is selected from the group consisting of botulinum toxin types A, B, C, D, E, F and G.
6. The method of the separate or combined embodiments 1-5, wherein the at least one hydrophilic polymer is selected from the group consisting of polysaccharides, hydroxyalkyl starch derivatives, polyvinyl acetates, polyvinyl pyrrolidones, polyethylene glycols, and a poloxamer.
7. The method of the separate or combined embodiments 1-6, wherein the at least one bioadhesive/mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.
8. The method of the separate or combined embodiments 1-7, wherein the at least one mucosal permeation enhancer is selected from the group consisting of diethylene glycol monoethyl ether, glyceryl monooleate, and a cell penetrating peptide.
9. The method of the separate or combined embodiments 1-8, wherein the at least one dissolution controller is selected from the group consisting of sugars, glycerin, and propylene glycol.
10. The method of the separate or combined embodiments 1-9, further comprising:
   prior to applying, coating the tip of a delivery device with the biodegradable film; and
   wherein said applying comprises inserting the delivery device into a nasal cavity of a subject and contacting a mucosal membrane of the subject with the film.
11. A biodegradable film, comprising:
   about 0.0056 to 560 ng of a therapeutically effective amount of a Clostridial toxin active ingredient;
   about 20 to 95% (w/w) of at least one polymer selected from a hydrophilic polymer and a bioadhesive/mucoadhesive polymer; and
   at least one of a mucosal permeation enhancer and at least one dissolution controller.
12. The film of embodiment 11, wherein the film comprises about 0.0001 to 15% (w/w) of the at least one mucosal permeation enhancer.
13. The film of the separate or combined embodiments 11-12, wherein the film comprises about 0.1 to 15 % (w/w) of the at least one dissolution controller.
14. The film of the separate or combined embodiments 11-13, comprising at least one mucosal permeation enhancer and at least one dissolution controller.
15. The film of the separate or combined embodiments 11-14, wherein the Clostridial toxin active ingredient is selected from the group consisting of botulinum toxin types A, B, C, D, E, F and G.
16. The film of the separate or combined embodiments 11-15, wherein the hydrophilic polymer is selected from the group consisting of polysaccharides, hydroxyalkyl starch derivatives, polyvinyl acetates, polyvinyl pyrrolidones, polyethylene glycols, and a poloxamer.
17. The film of the separate or combined embodiments 11-16, wherein the bioadhesive/mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.
18. The film of the separate or combined embodiments 11-17, wherein the at least one mucosal permeation enhancer is selected from the group consisting of diethylene glycol monoethyl ether, glyceryl monooleate, and a cell penetrating peptide.
19. The film of the separate or combined embodiments 11-18, wherein the dissolution controller is selected from the group consisting of sugars, glycerin, and propylene glycol.
20. A biodegradable film, comprising:
   a therapeutically effective amount of a Clostridial toxin active ingredient;
   at least one polymer selected from a hydrophilic polymer and a bioadhesive/mucoadhesive polymer;
   at least one mucosal permeation enhancer; and
   at least one dissolution controller.
21. The film of embodiment 20, wherein the film comprises about 0.0056 to 560 ng of the Clostridial toxin active ingredient.
22. The film of the separate or combined embodiments 20-21, wherein the Clostridial toxin active ingredient is selected from the group consisting of botulinum toxin types A, B, C, D, E, F and G.
23. The film of the separate or combined embodiments 20-22, wherein the film comprises about 20 to 95% (w/w) of the at least one polymer.
24. The film of the separate or combined embodiments 20-23, wherein the film comprises about 0.1 to 15 % (w/w) of the at least one dissolution controller.
25. The film of the separate or combined embodiments 20-24, wherein the at least one dissolution controller is selected from sugars, glycerin, and propylene glycol.
26. The film of the separate or combined embodiments 20-25, wherein at least one of the at least one polymers is a hydrophilic polymer.
27. The film of the separate or combined embodiments 20-26, wherein the hydrophilic polymer is selected from the group consisting of polysaccharides, hydroxyalkyl starch derivatives, polyvinyl acetates, polyvinyl pyrrolidones, polyethylene glycols, and a poloxamer.
28. The film of the separate or combined embodiments 20-27, wherein at least one of the at least one polymers is a bioadhesive and/or mucoadhesive polymer.
29. The film of the separate or combined embodiments 20-28, wherein the bioadhesive/mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.
30. The film of the separate or combined embodiments 20-29, wherein the film comprises about 0.0001 to 15% (w/w) of the at least one mucosal permeation enhancer.
31. The film of the separate or combined embodiments 20-30, wherein the at least one mucosal permeation enhancer is selected from the group consisting of diethylene glycol monoethyl ether, glyceryl monooleate, and a cell penetrating peptide.

While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions and sub-combinations as are within their true spirit and scope.
1. A method of treating rhinitis or a symptom of rhinitis, comprising:
   administering a biodegradable or biocompatible film to a nasal cavity of a subject thereby relieving one or more symptoms of rhinitis, the film comprising:
   a therapeutically effective amount of a Clostridial toxin active ingredient;
   at least one polymer selected from a hydrophilic polymer and a mucoadhesive polymer; and
      at least one of:
      (a) at least one mucosal permeation enhancer; and
      (b) at least one dissolution controller.
2. The method of statement 1, wherein the film dissolves at the administration site in less than about 10 minutes.
3. The method of statement 1 or 2, further comprising:
   prior to applying, coating the tip of a delivery device with the biodegradable film; and
   wherein said applying comprises inserting the delivery device into a nasal cavity of a subject and contacting a mucosal membrane of the subject with the film.
4. A biodegradable film, comprising:
   about 0.0056 to 560 ng of a therapeutically effective amount of a Clostridial toxin active ingredient;
   about 20 to 95% (w/w) of at least one polymer selected from a hydrophilic polymer and a mucoadhesive polymer; and
      at least one of a mucosal permeation enhancer and at least one dissolution controller.
5. The film of statement 41, wherein the film comprises about 0.0001 to 15% (w/w) of the at least one mucosal permeation enhancer.
6. The film of statement 4 or 5, wherein the film comprises about 0.1 to 15 % (w/w) of the at least one dissolution controller.
7. The film of any one of statements 4 to 6, wherein the hydrophilic polymer is selected from the group consisting of polysaccharides, hydroxyalkyl starch derivatives, polyvinyl acetates, polyvinyl pyrrolidones, polyethylene glycols, and a poloxamer.
8. The film of any one of statements 4 to 7, wherein the mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.
9. The film of any one of statements 4 to 8, wherein the at least one mucosal permeation enhancer is selected from the group consisting of diethvlene glycol monoethyl ether, glyceryl monooleate, and a cell penetrating peptide.
10. The film of any one of statements 4 to 9, wherein the dissolution controller is selected from the group consisting of sugars, glycerin, and propylene glycol.
11. A biodegradable film, comprising:
   a therapeutically effective amount of a Clostridial toxin active ingredient;
   at least one polymer selected from a hydrophilic polymer and a mucoadhesive polymer;
   at least one mucosal permeation enhancer; and
   at least one dissolution controller.
12. The film of statement 11, wherein the film comprises about 20 to 95% (w/w) of the at least one polymer.
13. The film of statement 11 or 12, wherein the film comprises about 0.1 to 15 % (w/w) of the at least one dissolution controller.
14. The film of any one of statements 11 to 13, wherein the at least one dissolution controller is selected from sugars, glycerin, and propylene glycol.
15. The film of any one of statements 11 to 14, wherein at least one of the at least one polymers is a hydrophilic polymer.
16. The film of statement 15, wherein the hydrophilic polymer is selected from the group consisting of polysaccharides, hydroxyalkyl starch derivatives, polyvinyl acetates, polyvinyl pyrrolidones, polyethylene glycols, and a poloxamer.
17. The film of any one of statements 11 to 16, wherein at least one of the at least one polymers is a mucoadhesive polymer.
18. The film of statement 17, wherein the mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.
19. The film of any one of statements 11 to 18, wherein the film comprises about 0.0001 to 15% (w/w) of the at least one mucosal permeation enhancer.
20. The film of any one of statements 11 to 19, wherein the at least one mucosal permeation enhancer is selected from the group consisting of diethylene glycol monoethyl ether, glyceryl monooleate, and a cell penetrating peptide.

## Claims

1. A biodegradable or biocompatible film for use in a method of treating rhinitis or a symptom of rhinitis, comprising:
administering the biodegradable or biocompatible film to a nasal cavity of a subject thereby relieving one or more symptoms of rhinitis, the film comprising:
a therapeutically effective amount of a Clostridial toxin active ingredient; at least one polymer selected from a hydrophilic polymer and a mucoadhesive polymer; and at least one of:
(a) at least one mucosal permeation enhancer; and
(b) at least one dissolution controller.

2. The film for use of claim 1, wherein the film dissolves at the administration site in less than 10 minutes.

3. The film for use of claim 1 or 2, further comprising:
prior to applying, coating the tip of a delivery device with the biodegradable film; and
wherein said applying comprises inserting the delivery device into a nasal cavity of a subject and contacting a mucosal membrane of the subject with the film.

4. A biodegradable film, comprising:
0.0056 to 560 ng of a therapeutically effective amount of a Clostridial toxin active ingredient;
20 to 95% (w/w) of at least one polymer selected from a hydrophilic polymer and a mucoadhesive polymer; and
at least one of a mucosal permeation enhancer and at least one dissolution controller.

5. The film of claim 4, wherein the film comprises 0.0001 to 15% (w/w) of the at least one mucosal permeation enhancer.

6. The film of claim 4 or 5, wherein the film comprises 0.1 to 15 % (w/w) of the at least one dissolution controller.

7. The film of any one of claims 4 to 6, wherein:
(i) the hydrophilic polymer is selected from the group consisting of polysaccharides, hydroxyalkyl starch derivatives, polyvinyl acetates, polyvinyl pyrrolidones, polyethylene glycols, and a poloxamer; and/or
(ii) the mucoadhesive polymer is selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers; and/or
(iii) the at least one mucosal permeation enhancer is selected from the group consisting of diethvlene glycol monoethyl ether, glyceryl monooleate, and a cell penetrating peptide; and/or
(iv) the dissolution controller is selected from the group consisting of sugars, glycerin, and propylene glycol.

8. A biodegradable film, comprising:
a therapeutically effective amount of a Clostridial toxin active ingredient;
at least one polymer selected from a hydrophilic polymer and a mucoadhesive polymer;
at least one mucosal permeation enhancer; and
at least one dissolution controller.

9. The film of claim 8, wherein the film comprises 20 to 95% (w/w) of the at least one polymer.

10. The film of claim 8 or 9, wherein the film comprises 0.1 to 15 % (w/w) of the at least one dissolution controller.

11. The film of any one of claims 8 to 10, wherein the at least one dissolution controller is selected from sugars, glycerin, and propylene glycol.

12. The film of any one of claims 8 to 11, wherein at least one of the at least one polymers is a hydrophilic polymer, optionally selected from the group consisting of polysaccharides, hydroxyalkyl starch derivatives, polyvinyl acetates, polyvinyl pyrrolidones, polyethylene glycols, and a poloxamer.

13. The film of any one of claims 8 to 12, wherein at least one of the at least one polymers is a mucoadhesive polymer, optionally selected from the group consisting of polyacrylic acids, polyacrylates, poly(meth)acrylates, poly(ethylene oxide), pectin, sodium alginate, carrageenan, cellulose derivatives, hyaluronic acid, chitosan, alginates, pectin, gelatin, and poloxamers.

14. The film of any one of claims 8 to 13, wherein the film comprises 0.0001 to 15% (w/w) of the at least one mucosal permeation enhancer.

15. The film of any one of claims 8 to 14, wherein the at least one mucosal permeation enhancer is selected from the group consisting of diethylene glycol monoethyl ether, glyceryl monooleate, and a cell penetrating peptide.
